(19) Europäisches Patentamt
European Patent Office
Office européen des brevets

(11) Numéro de publication: **0 306 375**
**A1**

(12) # DEMANDE DE BREVET EUROPEEN

(21) Numéro de dépôt: **88402022.3**

(22) Date de dépôt: **03.08.88**

(51) Int. Cl.[4]: **C 07 D 401/06**
C 07 D 401/14,
C 07 D 405/14,
C 07 D 409/14, A 61 K 31/47

(30) Priorité: **07.08.87 FR 8711288**
**30.12.87 FR 8718342**
**19.04.88 FR 8805129**
**19.04.88 FR 8805130**

(43) Date de publication de la demande:
**08.03.89 Bulletin 89/10**

(84) Etats contractants désignés:
**AT BE CH DE ES FR GB GR IT LI LU NL SE**

(71) Demandeur: **SYNTHELABO**
**58 rue de la Glacière**
**F-75013 Paris (FR)**

(72) Inventeur: **George, Pascal**
**39, rue Henri de Vilmorin**
**F-94400 Vitry sur Seine (FR)**

**Sevrin, Mireille**
**73, rue Raymond Losserand**
**F-75015 Paris (FR)**

**Maloizel, Christian**
**21, rue du Plateau**
**F-92190 Meudon (FR)**

(74) Mandataire: **Ludwig, Jacques et al**
**SYNTHELABO Service Brevets 58, rue de la Glacière**
**F-75013 Paris (FR)**

Claims for the following Contracting States: ES + GR.

(54) **Dérivés de[(pipéridinyl-4)méthyl]-2 tétrahydro-1,2,3,4 isoquinoléine, leur préparation et leur application en thérapeutique.**

(57) Composés répondant à la formule générale (I)

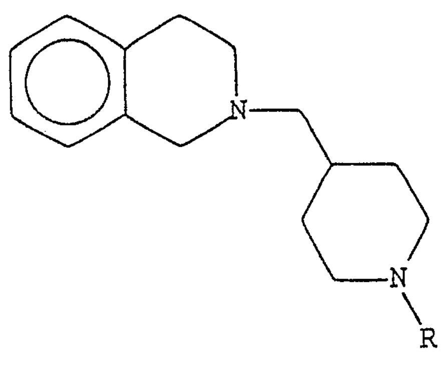

(I)

dans laquelle R représente l'hydrogène ou un groupe alkyle, allyle, cycloalkylméthyle, phénylméthyle éventuellement substitué, phényl-2 éthyle, phényl-3 propyle, phényl-3 propène-2 yle, phénylcarbonylméthyle, naphtylméthyle, pyridinylméthyle, furannylméthyle, thiénylméthyle, alcanoyle, cycloalkylcarbonyle, trifluoroacétyle, phénylcarbonyle éventuellement substitué, oxo-1 phényl-3 propène-2 yle, naphtylcarbonyle, pyridinylcarbonyle, furannylcarbonyle, thiénylcarbonyle ou indolylcarbonyle.
Application en thérapeutique.

EP 0 306 375 A1

**Description**

## DERIVES DE [(PIPERIDINYL-4)METHYL]-2 TETRAHYDRO-1,2,3,4 ISOQUINOLEINE, LEUR PREPARATION ET LEUR APPLICATION EN THERAPEUTIQUE

La présente invention a pour objet des dérivés de [(pipéridinyl-4)méthyl]-2 tétrahydro-1,2,3,4 isoquinoléine, leur préparation et leur application en thérapeutique.

Les composés de l'invention répondent à la formule générale (I) donnée dans le schéma de la page suivante, formule dans laquelle R représente

a) soit un atome d'hydrogène ;

b) soit un groupe $(C_1-C_6)$alkyle linéaire ou ramifié ; un groupe allyle ; un groupe $(C_3-C_6)$cycloalkylméthyle ; un groupe phénylméthyle portant éventuellement de un à trois substituants choisis parmi les atomes d'halogènes et les groupes trifluorométhyle, nitro, amino, diméthylamino, cyano, aminocarbonyle, $(C_1-C_3)$alkyle linéaires ou ramifiés, $(C_1-C_3)$alcoxy linéaires ou ramifiés, $(C_1-C_3)$alkylthio linéaires ou ramifiés ; un groupe phényl-2 éthyle ; un groupe phényl-3 propyle ; un groupe phényl-3 propène-2 yle ; un groupe phénylcarbonylméthyle ; un groupe naphtylméthyle ; un groupe pyridinylméthyle ; un groupe furannylméthyle ; ou un groupe thiénylméthyle.

c) soit un groupe $(C_2-C_6)$alcanoyle linéaire ou ramifié ; un groupe $(C_3-C_6)$cycloalkylcarbonyle ; un groupe trifluoroacétyle ; un groupe phénylcarbonyle portant éventuellement de un à trois substituants choisis parmi les atomes d'halogènes et les groupes trifluorométhyle, nitro, $(C_1-C_3)$alkyle linéaires ou ramifiés, $(C_1-C_3)$alcoxy linéaires ou ramifiés, $(C_1-C_3)$alkylthio linéaires ou ramifiés ; un groupe oxo-1 phényl-3 propène-2 yle ; un groupe naphtylcarbonyle ; un groupe pyridinylcarbonyle ; un groupe furannylcarbonyle ; un groupe thiénylcarbonyle ; un groupe (indolyl-2)carbonyle ; ou un groupe (indolyl-5)carbonyle.

Ils peuvent se présenter à l'état de bases libres ou de sels d'addition à des acides acceptables en pharmacologie.

Dans la description qui suit les substituants R définis cidessus selon b)seront appelés "groupes réduits", et ceux définis ci-dessus selon c) seront appelés "groupes acyles". Conformément à l'invention on peut préparer les composés de

Schéma 1

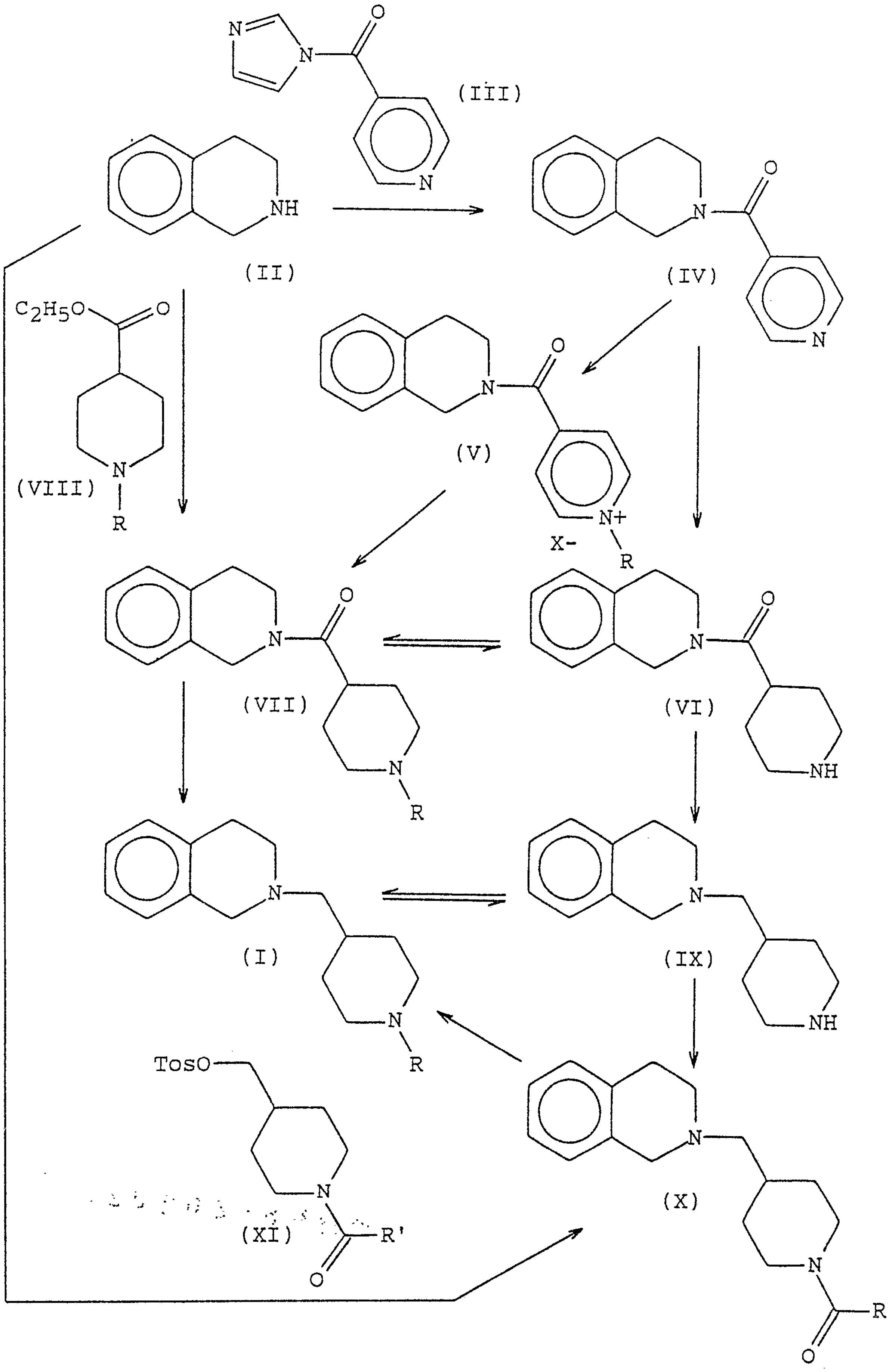

(III) (II) (IV) (V) (VIII) (VII) (VI) (I) (IX) (XI) (X)

Schéma 2

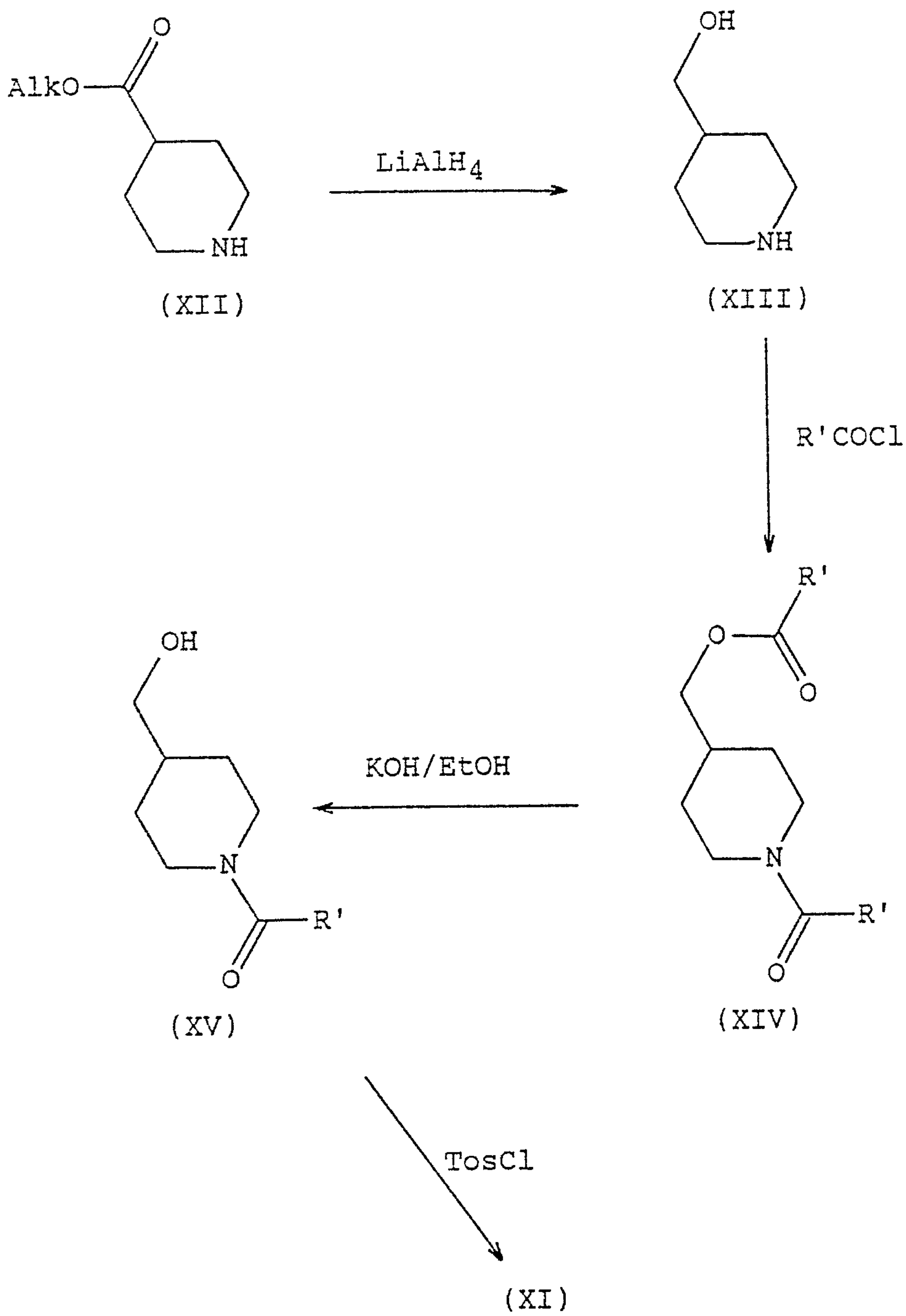


formule générale (I) par un procédé illustré par le schéma 1 qui précède.

On fait réagir la tétrahydro-1,2,3,4 isoquinoléine de formule (II) avec l'imidazolide de formule (III) (préparé in situ au moyen d'acide isonicotinique et de N,N'-carbonyldiimidazole), pour obtenir l'amide de formule (IV).

Ensuite on soumet l'amide (IV) à une hydrogénation catalytique, donnant l'amide de formule (VI) (comme décrit dans le brevet des Etats Unis d'Amérique N°4243666), qu'on réduit au moyen d'hydrure de lithium et d'aluminium ou d'un hydrure simple ou complexe de bore, tel que le diborane ou le complexe de borane/sulfure de méthyle, si l'on veut préparer le composé de formule (IX), c'est-à-dire le composé de formule générale (I) où R représente un atome d'hydrogène, ou bien on soumet l'amide (VI) à une alkylation au moyen d'un halogénure de formule générale RX (R étant un groupe "réduit", et X étant un groupe labile, par exemple un atome de brome), si l'on veut préparer un composé de formule générale (I) où R est un groupe "réduit", et finalement on réduit l'amide (VII) ainsi obtenu de la manière décrite à propos de l'amide (VI).

On peut aussi obtenir l'amide intermédiaire de formule générale (VII) en alkylant d'abord l'amide de formule (IV), pour obtenir l'amide de formule générale (V), et soumettre ensuite ce dernier à l'hydrogénation catalytique. Une autre variante du procédé selon l'invention consiste à préparer l'amide de formule générale (VII) en faisant réagir la tétrahydro-1,2,3,4 isoquinoléine de formule (II) avec un isonipécotate de formule générale (VIII) (dans laquelle R est un groupe "réduit"), en présence de triméthylaluminium. Il va de soi qu'on peut encore passer de l'amide de formule générale (VII), où R représente un groupe benzyle, à l'amide non substitué de formule (VI) par une hydrogénolyse en présence de palladium.

De la même manière on peut passer du composé de formule générale (I), où R représente un groupe benzyle, à l'amine non substituée de formule (IX).

Inversement, on peut passer de l'amine de formule (IX) à une amine de formule générale (I) où R est un groupe "réduit" par une alkylation au moyen d'un halogénure de formule générale RX (R étant un tel groupe "réduit" et X un groupe labile). Un composé de formule générale (I) dans laquelle R est un groupe "acyle" peut être obtenu par réaction du composé de formule (IX) avec un dérivé de formule générale R'COY, dans laquelle R'CO correspond à un groupe R "acyle", et Y représente un groupe labile tel qu'un atome d'halogène, par exemple le chlore, ou tel qu'un groupe imidazolyle-1, ou tel qu'un groupe alcoxy en $C_1$-$C_6$, ou tel qu'un groupe acyloxy de formule $R'CO_2$.

Lorsque Y représente un atome d'halogène, on fait réagir le composé de formule (IX) avec l'halogénure d'acide de formule générale R'COY, en présence d'une base organique, par exemple la triéthylamine, dans un solvant tel que le dichlorométhane ou l'acétate d'éthyle.

Lorsque Y représente un groupe imidazolyle-1, on fait réagir le composé de formule (IX) avec l'imidazolide de formule générale R'COY (préparé in situ au moyen de N,N'-carbonyldiimidazole et de l'acide correspondant de formule générale R'COOH), dans un solvant tel que le tétrahydrofuranne. Lorsque Y représente un groupe alcoxy, on fait réagir le composé de formule (IX) avec un trialkylaluminium, par exemple le triméthylaluminium, puis on fait réagir le complexe ainsi formé avec l'ester de formule générale R'COY, à une température de 50 à 110°C, dans un solvant inerte aromatique, par exemple le toluène. Lorsque Y représente un groupe acyloxy, on fait réagir le composé de formule (IX) avec l'anhydride de formule générale $(R'CO)_2$, soit avec ledit anhydride comme solvant, soit dans un solvant inerte à une température de 0 à 60°C. Dans tous les cas on obtient donc un amide de formule générale (X).

Ce dernier peut encore être obtenu en une seule étape, qui consiste à faire réagir la tétrahydro-1,2,3,4 isoquinoléine de formule (II) avec un tosylate de formule générale (XI) (dans laquelle Tos représente un groupe tosyle et R'CO représente un groupe R "acyle"), soit en l'absence soit en présence d'un solvant inerte tel que le diméthylformamide ou le xylène, à une température de 20 à 150°C, et éventuellement en présence d'une base organique telle qu'une amine tertiaire, ou minérale telle qu'un carbonate ou hydrogénocarbonate alcalin.

Le tosylate de formule générale (XI) peut être préparé selon une méthode illustrée par le schéma 2 qui précède. On réduit un pipéridine-4-carboxylate de formule générale (XII) (dans laquelle Alk représente un groupe alkyle inférieur), par exemple au moyen d'hydrure de lithium et aluminium, pour obtenir le pipéridine-4-méthanol de formule (XIII), et on fait réagir ce dernier avec un chlorure d'acide de formule générale R'COCl (dans laquelle R'CO représente un groupe R "acyle"), dans un solvant inerte tel qu'un solvant chloré, à une température de 20 à 80°C. On obtient ainsi un ester-amide de formule générale (XIV), qu'on saponifie , par exemple au moyen d'hydroxyde de sodium ou de potassium, dans un solvant alcoolique aliphatique inférieur, de préférence l'éthanol, pour obtenir l'alcool de formule générale (XV), dont on prépare finalement le tosylate au moyen de chlorure de tosyle, dans un milieu basique tel que la pyridine. Les amides de formule générale (X) peuvent ensuite, si on le désire, être réduits en amines de formule générale (I) où R représente un groupe "réduit" au moyen d'un hydrure simple ou complexe de bore, tel que le diborane ou le complexe de borane/sulfure de méthyle, l'hydrure de lithium et d'aluminium, l'hydrure d'aluminium, etc, dans un solvant éthéré tel que l'éther diéthylique, le tétrahydrofuranne ou le dioxanne, à une température de 20 à 100°C.

Selon les substituants présents dans le groupe R, il va de soi que l'on peut transformer, par des procédés bien connus, certains composés de formule générale (I) en d'autres composés de formule générale (I), en agissant sur lesdits substituants.

Ainsi, par exemple, un composé de formule générale (I), dans laquelle R représente un groupe benzyle portant un groupe aminocarbonyle, peut être obtenu à partir d'un composé de formule générale (I), dans laquelle R représente un groupe benzyle portant un groupe cyano, par réaction de ce dernier avec l'acide chlorhydrique gazeux en présence d'acide formique.

Un composé de formule générale (I), dans laquelle R représente un groupe benzyle portant un groupe

amino, peut etre obtenu à partir d'un composé de formule générale (I), dans laquelle R représente un groupe benzyle portant un groupe nitro, par réduction de ce dernier au moyen de diborane ou de complexe diborane/sulfure de méthyle, dans un solvant éthéré tel que le tétrahydrofuranne.

Un composé de formule générale (I) dans laquelle R représente un groupe benzyle portant un groupe diméthylamino, peut être obtenu à partir d'un composé de formule générale (I), dans laquelle R représente un groupe benzyle portant un groupe amino, par amination réductrice de ce dernier, c'est-à-dire par réaction avec le formaldéhyde en présence d'un acide tel que l'acide sulfurique, dans un solvant éthéré tel que le tétrahydrofuranne, puis par réduction de l'adduit ainsi obtenu au moyen de borohydrure de sodium.

Les exemples qui suivent illustrent en détail la préparation de quelques composés selon l'invention. Les microanalyses élémentaires et les spectres IR et RMN confirment les structures des produits obtenus.

Les numéros donnés entre parenthèses dans les titres des exemples correspondent à ceux du tableau donné plus loin.

Exemple 1 (Composé N°5)

[[[(Chloro-3 phényl)méthyl]-1 pipéridinyl-4]méthyl]-2 tétrahydro-1,2,3,4- isoquinoléine, difumarate.

a) [(Pyridinyl-4)carbonyl]-2 tétrahydro-1,2,3,4 isoquinoléine.

Sous atmosphère d'argon on dissout 50 g (406 mmoles) d'acide isonicotinique dans 1000 ml de tétrahydrofuranne et, tout en agitant, on ajoute 25 g de N,N'-carbonyldiimidazole, on agite une demi-heure, on ajoute encore 25 g de N,N'-carbonyldiimidazole, on agite encore une demi-heure et on ajoute 15 g de N,N'-carbonyldiimidazole. On maintient l'agitation pendant 1h30 et, dans le mélange devenu limpide, on introduit lentement 51,39 g (385,8 mmoles) de tétrahydro-1,2,3,4 isoquinoléine dissoute dans quelques millilitres de tétrahydrofuranne, et on agite le mélange pendant une nuit à température ambiante. Ensuite on évapore le solvant, on reprend le résidu huileux avec environ 700 ml de dichlorométhane, on lave la solution obtenue deux fois avec une solution saturée de bicarbonate de sodium, puis deux fois avec de l'eau et on la sèche sur sulfate de magnésium. On obtient 87,48 g d'une huile orangée qui se solidifie lentement à froid.

b) [(Pipéridinyl-4)carbonyl]-2 tétrahydro-1,2,3,4 isoquinoléine.

On effectue une hydrogénation catalytique de 35 g (127,3 mmoles) de chlorhydrate de [(pyridinyl-4)carbonyl]-2 tétrahydro-1,2,3,4 isoquinoléine dans 600 ml de méthanol, en présence de 1,2 g d'oxyde de platine, pendant 17h, sous une pression d'hydrogène d'environ 0,38 MPa. Puis on filtre le catalyseur, on évapore le méthanol, on reprend le résidu avec de l'éther, et on filtre l'insoluble. On obtient 33,7 g de chlorhydrate dont on libère la base en l'introduisant dans un mélange d'eau et de dichlorométhane en ajoutant du carbonate de potassium par portions jusqu'à pH > 10. On sépare la phase organique, on la sèche sur sulfate de sodium et on évapore le solvant. On recristallise le résidu dans l'éther, on le filtre, on le lave avec du pentane et on le sèche. On obtient 17,2 g de base. Point de fusion : 200-205°C (décomposition).

c) [[[(Chloro-3 phényl)méthyl]-1 pipéridinyl-4]carbonyl]-2 tétrahydro-1,2,3,4 isoquinoléine.

Dans 100 ml d'acétone anhydre on introduit 5,2 g (21,3 mmoles) de [(pipéridinyl-4)carbonyl]-2 tétrahydro-1,2,3,4 isoquinoléine, 4,94 g (25,5 mmoles) de bromo-1 chlorométhyl-3 benzène et 4,71 g (34 mmoles) de carbonate de potassium. On agite le mélange pendant une nuit à température ambiante, puis on évapore l'acétone, on reprend le résidu avec du dichlorométhane et de l'eau, on sépare la phase organique, on la lave, on la sèche et on évapore le solvant. On obtient 10,5 g de produit qu'on purifie par chromatographie sur colonne de silice, d'abord avec du dichlorométhane, puis avec un mélange 97/3 de dichlorométhane/méthanol. On reprend le produit purifié avec du pentane, et on évapore ce dernier. On obtient 6,13 g de base pure. Point de fusion : 114-115°C.

d) [[[(Chloro-3 phényl)méthyl]-1 pipéridinyl-4]méthyl]-2 tétrahydro-1,2,3,4 isoquinoléine, difumarate.

On dissout 4,5 g (12,2 mmoles) de [[[(chloro-3 phényl)méthyl]-1 pipéridinyl-4]carbonyl]-2 tétrahydro-1,2,3,4 isoquinoléine dans 70 ml de tétrahydrofuranne, on ajoute 3,66 ml (36,6 mmoles) d'une solution 10N de complexe de borane/sulfure de méthyle, on chauffe le mélange à 50°C pendant 2h, et on l'agite pendant 2 jours à température ambiante. Ensuite on hydrolyse le mélange avec 20 ml de méthanol puis 10 ml d'acide chlorhydrique concentré, on le chauffe au reflux pendant 3h et on le laisse au repos pendant une nuit. On évapore le solvant, on reprend le résidu avec de l'éther et de l'eau, on sépare la phase aqueuse, on y ajoute du bicarbonate de sodium aqueux à 30% jusqu'à pH > 10 et on l'extrait avec du dichlorométhane. On sépare la phase organique, on la lave avec de l'eau, on la sèche sur sulfate de sodium et on évapore le solvant. On obtient 4,5 g d'une huile claire qu'on purifie par chromatographie sur colonne de silice en éluant avec un mélange 98/2 de dichlorométhane/ méthanol. On obtient 3,8 g d'huile qu'on dissout dans de l'acétate d'éthyle

et on verse cette solution goutte à goutte dans une solution d'acide fumarique dans le minimum de méthanol. On filtre le sel précipité, on le lave à l'acétate d'éthyle, à l'acétone et à l'éther et on le sèche. On obtient 3,9 g de difumarate. Point de fusion : 188-190°C (décomposition).

Exemple 2 (Composé N°3)

[(Phénylméthyl-1 pipéridinyl-4)méthyl]-2 tétrahydro-1,2,3,4 isoquinoléine, dichlorhydrate.

a) [(Phénylméthyl-1 pipéridinyl-4)carbonyl]-2 tétrahydro-1,2,3,4 isoquinoléine.

Sous atmosphère d'argon on introduit 251 ml (594 mmoles) de triméthylaluminium (à 25% dans l'hexane) dans 400 ml de toluène. On refroidit la solution dans un bain de glace et on ajoute 81,5 g (612 mmoles) de tétrahydro-1,2,3,4 isoquinoléine en solution dans 200 ml de toluène. On chauffe à environ 50°C et on ajoute 95 g (384 mmoles) de phénylméthyl-1 pipéridinyl-4-carboxylate d'éthyle en solution dans 400 ml de toluène. On chauffe le mélange au reflux pendant 2h en éliminant environ 250 ml de solvant à l'aide d'un appareil de Dean-Stark. On refroidit le mélange par un bain de glace, on l'hydrolyse en ajoutant lentement 250 ml d'eau et on filtre l'insoluble, en le rinçant avec de l'acétate d'éthyle. On lave le filtrat trois fois avec de l'eau, on sèche la phase organique sur sulfate de sodium, on évapore le solvant et on recristallise le résidu dans du cyclohexane. On obtient 116,6 g de produit. Point de fusion : 98-100°C.

b) [(Phénylméthyl-1 pipéridinyl-4)méthyl]-2 tétrahydro-1,2,3,4 isoquinoléine, dichlorhydrate.

- Première variante.

Sous atmosphère d'argon on introduit 8 g (24 mmoles) de [(phénylméthyl-1 pipéridinyl-4)carbonyl]-2 tétrahydro-1,2,3,4 isoquinoléine dans 150 ml de tétrahydrofuranne, on ajoute 5 g (133 mmoles de borohydrure de sodium, on refroidit le mélange à -10°C et on ajoute 19 ml (155 mmoles) de complexe de trifluorure de bore/éther et on agite pendant 1h à -10°C. On hydrolyse le mélange à 0°C avec 66 ml d'acide chlorhydrique 1N, on le neutralise avec de l'ammoniaque concentrée et on l'extrait avec du dichlorométhane. On sépare la phase organique, on la lave deux fois à l'eau, on la sèche et on évapore le solvant. On reprend le résidu avec 200 ml d'éthanol, on y fait passer un courant d'acide chlorhydrique gazeux et on chauffe au reflux pendant 6h. On évapore le solvant, on reprend le résidu avec de l'éther et de l'eau, on ajoute de l'ammoniaque concentrée, on sépare la phase organique, on la lave à l'eau, on la sèche et on évapore le solvant. On obtient 6,9 g de produit huileux qu'on dissout dans de l'alcool isopropylique, on y fait passer un courant d'acide chlorhydrique gazeux et on recristallise le sel dans l'éthanol. On obtient 7,8 g de dichlorhydrate. Point de fusion : 260-265°C.

- Deuxième variante.

Sous atmosphère d'argon on introduit 60 g (179 mmoles) de [(phénylméthyl-1 pipéridinyl-4)carbonyl]-2 tétrahydro-1,2,3,4 isoquinoléine par petites portions dans une suspension de 10,2 g (268 mmoles) d'hydrure de lithium et d'aluminium dans 3 1 d'éther chauffé au reflux. On maintient l'agitation pendant 2h, puis on refroidit le mélange par un bain de glace et on l'hydrolyse avec 24,12 ml de soude 1N. On filtre l'insoluble, on concentre le filtrat, on reprend le résidu dans de l'éther et on y fait passer un courant de gaz chlorhydrique. On filtre le sel qui précipite et on le sèche. On obtient 66,7 g de dichlorhydrate. Point de fusion : 259-264°C.

Exemple 3 (Composé N°1)

[(Pipéridinyl-4)méthyl]-2 tétrahydro-1,2,3,4 isoquinoléine, dichlorhydrate

a) Premiére variante

Sous atmosphère d'argon on ajoute 5,8 g (23,7 mmoles) de [(pipéridinyl-4)carbonyl]-2 tétrahydro-1,2,3,4 isoquinoléine à une solution de 0,9 g (23,7 mmoles) d'hydrure de lithium et d'aluminium dans 150 ml d'éther. On chauffe le mélange au reflux pendant 7h. On le refroidit, on l'hydrolyse avec 2 ml d'eau, on filtre l'insoluble, on sépare la phase organique, on la sèche, on évapore le solvant, on reprend le résidu dans un mélange d'alcool isopropylique et de méthanol et on y fait passer un courant d'acide chlorhydrique gazeux. On obtient ainsi 3,7 g de dichlorhydrate. Point de fusion : 252-255°C.

) Deuxième variante.

On soumet à une hydrogénation catalytique un mélange de 11,2 g (28,4 mmoles) de dichlorhydrate de [(phénylméthyl-1 pipéridinyl-4)méthyl]-2 tétrahydro-1,2,3,4 isoquinoléine, 1,5 g de charbon palladié à 10% et 200 ml d'éthanol, sous une pression d'hydrogène d'environ 0,41 MPa et à une température de 40° C. On filtre le catalyseur en le rinçant avec de l'éthanol, on évapore le filtrat et on recristallise le résidu dans l'éthanol. On obtient 6,9 g de dichlorhydrate. Point de fusion : 252-255° C.

Exemple 4 (Composé N°2)

[(Méthyl-1 pipéridinyl-4)méthyl]-2 tétrahydro-1,2,3,4 isoquinoléine, difumarate.

a) Iodure de méthyl-1 [(tétrahydro-1,2,3,4 isoquinoléinyl-2)carbonyl]-4 pyridinium.

On introduit 2 ml, soit 4,26 g (30,2 mmoles) d'iodométhane et 6 g (25,2 mmoles) de [(pyridinyl-4)carbonyl]-2 tétrahydro-1,2,3,4 isoquinoléine dans 200 ml d'acétate d'éthyle, et on agite le mélange pendant 24h à température ambiante. Il se forme un précipité jaune qu'on filtre et lave à l'éther. On obtient 8,3 g de produit qu'on utilise tel quel dans l'étape suivante.

b) [(Méthyl-1 pipéridinyl-4)carbonyl]-2 tétrahydro-1,2,3,4 isoquinoléine.

On introduit 4 g (10,5 mmoles) d'iodure de méthyl-1 [(tétrahydro-1,2,3,4 isoquinoléinyl-2)carbonyl]-4 pyridinium et 0,4 g d'oxyde de platine dans 250 ml de méthanol et on soumet le mélange à hydrogénation pendant 12h sous une pression d'environ 0,41 MPa. On filtre le catalyseur, on évapore le solvant, on neutralise le résidu et on obtient une huile qui cristallise et qu'on utilise telle quelle dans l'étape suivante.

c) [(Méthyl-1 pipéridinyl-4)méthyl]-2 tétrahydro-1,2,3,4 isoquinoléine, difumarate.

Sous atmosphère d'argon on dissout 3 g (11,6 mmoles) de [(méthyl-1 pipéridinyl-4)carbonyl]-2 tétrahydro-1,2,3,4 isoquinoléine dans 100 ml de tétrahydrofuranne, on ajoute 0,9 g d'hydrure de lithium et d'aluminium et on chauffe le mélange au reflux pendant 4h. On continue d'agiter pendant 12h à température ambiante, puis on hydrolyse le mélange avec de la soude, on sépare la phase organique et on l'évapore On obtient une huile jaune dont on prépare le difumarate. Point de fusion : 157-158° C.

Exemple 5 (Composé N° 14)

[(Phénylcarbonyl-1 pipéridinyl-4)méthyl]-2 tétrahydro-1,2,3,4 isoquinoléine, chlorhydrate.

a) [(Pipéridinyl-4)carbonyl]-2 tétrahydro-1,2,3,4 isoquinoléine.

On dissout 115 g (0,34 mole) de [(phénylméthyl-1 pipéridinyl-4)carbonyl]-2 tétrahydro-1,2,3,4 isoquinoléine dans 1 l d'acide acétique, on ajoute 4 g de charbon palladié à 10% et on soumet le mélange à une hydrogénation à 75° C sous 0,35 MPa.

Lorsque l'absorption d'hydrogène est terminée, on laisse revenir le mélange à température ambiante, on filtre le catalyseur et on concentre le filtrat sous pression réduite. On reprend le résidu avec de l'eau, on le refroidit avec un bain de glace, on y ajoute de l'hydroxyde de sodium jusqu'à pH = 10 et on l'extrait avec du dichlorométhane. On lave la phase organique à l'eau, on la sèche sur sulfate de sodium, on évapore le solvant et on fait cristalliser le résidu dans de l'éther sec. On filtre les cristaux, on les lave avec du pentane et on les sèche sous vide. On obtient 73,5 g de produit. Point de fusion : 201-205° C (décomposition lente).

b) [(Pipéridinyl-4)méthyl]-2 tétrahydro-1,2,3,4 isoquinoléine, dichlorhydrate.

Sous atmosphère d'argon on ajoute 5,8 g (23,7 mmoles) de [(pipéridinyl-4)carbonyl]-2 tétrahydro-1,2,3,4 isoquinoléine à une suspension de 0,9 g (23,7 mmoles) d'hydrure de lithium et d'aluminium dans 150 ml d'éther. On chauffe le mélange au reflux pendant 7h. On le refroidit, on l'hydrolyse avec 2 ml d'eau, on filtre l'insoluble, on sépare la phase organique, on la sèche, on évapore le solvant, on reprend le résidu dans un mélange d'alcool isopropylique et de méthanol et on y fait passer un courant d'acide chlorhydrique gazeux. On obtient ainsi 3,7 g de dichlorhydrate. Point de fusion : 252-255° C.

c) [(Phénylcarbonyl-1 pipéridinyl-4)méthyl]-2 tétrahydro-1,2,3,4 isoquinoléine, chlorhydrate.

A une solution de 2,68 g (0,022 mole) d'acide benzoïque dans 75 ml de tétrahydrofuranne, placée sous atmosphère d'argon, on ajoute, par petites portions, 3,9 g (0,024 mole) de N,N'-carbonyldiimidazole. On plonge le réacteur dans un bain d'eau tiède et on agite le mélange pendant 1h.

Ensuite on ajoute 4,6 g (0,02 mole) de [(pipéridinyl-4) méthyl]-2 tétrahydro-1,2,3,4 isoquinoléine en solution dans 75 ml de tétrahydrofuranne, on agite le mélange pendant 6h à température ambiante puis pendant 9h au reflux.

On verse le mélange sur de l'eau glacée, on l'extrait avec du dichlorométhane, on sépare la phase organique et on la lave à l'ammoniaque puis à l'eau, on la sèche sur sulfate de magnésium et on évapore le solvant sous pression réduite.

On obtient 6,2 g de produit huileux qu'on purifie par chromatographie sur colonne de silice en éluant avec un mélange 98/2 de dichlorométhane/méthanol. On dissout la base dans un peu d'alcool isopropylique, on y fait passer un courant d'acide chlorhydrique gazeux, on filtre le chlorhydrate qui précipite, et on le recristallise trois fois dans de l'éthanol. On isole finalement 3,0 g de chlorhydrate. Point de fusion : 228-235°C (décomposition).

Exemple 6 (Composé N°16)

[[[(Chloro-3 phényl)carbonyl]-1 pipéridinyl-4]méthyl]-2 tétrahydro-1,2,3,4 isoquinoléine, chlorhydrate.

On introduit dans un ballon 4,6 g (0,02 mole) de [(pipéridinyl-4)méthyl]-2 tétrahydro-1,2,3,4 isoquinoléine, 2 g, soit 2,8 ml (0,02 mole) de triéthylamine et 100 ml d'acétate d'éthyle. En maintenant la température à environ 20°C à l'aide d'un bain froid, on ajoute alors, goutte à goutte, 3,5 g, soit 2,6 ml (0,02 mole) de chlorure de chloro-3 benzoyle en solution dans 50 ml d'acétate d'éthyle. On agite le mélange pendant 2h, on filtre le précipité, on le lave à l'acétate d'éthyle, on lave le filtrat à l'eau, on le sèche et on évapore le solvant.

On obtient 6,8 g de résidu huileux qu'on redissout dans l'acétate d'éthyle, on ajoute de l'éther chlorhydrique, on agite le mélange pendant 20 mn, on essore le chlorhydrate, on le lave à l'éther et on le recristallise dans un mélange 50/50 d'alcool isopropylique/acétate d'éthyle. On isole finalement 6,5 g de cristaux blancs. Point de fusion : 162-163°C.

Exemple 7 (Composé N°19)

[[[(Méthyl-3 phényl)carbonyl]-1 pipéridinyl-4]méthyl]-2 tétrahydro-1,2,3,4 isoquinoléine, fumarate.

On dissout 4,6 g (0,02 mole) de [pipéridinyl-4)méthyl]-2 tétrahydro-1,2,3,4 isoquinoléine dans 50 ml de dichlorométhane et on ajoute 2,23 g, soit 3,1 ml (0,022 mole) de triéthylamine puis, en 20 mn et au goutte à goutte,on ajoute une solution de 3,4 g (0,022 mole) de chlorure de méthyl-3 benzoyle en solution dans 10 ml de dichlorométhane. La température du mélange monte jusqu'à 38°C. On l'agite à température ambiante pendant une nuit, puis on le verse dans de l'eau, on l'extrait avec du dichlorométhane, on lave la phase organique quatre fois avec une solution saturée de chlorure de sodium, on la sèche sur sulfate de sodium et on évapore le solvant sous pression réduite. On obtient 7,8 g de résidu huileux brun qu'on purifie par chromatographie sur colonne de silice en éluant avec un mélange 90/10 de dichlorométhane/méthanol, ce qui fournit 6,9 g de produit purifié.

On en dissout 3,45 g dans 50 ml d'éthanol et on y ajoute une solution de 1,05 g d'acide fumarique dans 100 ml d'éthanol. On agite le mélange pendant 30 mn puis on évapore le solvant sous pression réduite, on reprend le résidu avec 250 ml d'acétone, on laisse cristalliser au froid, on filtre les cristaux et on les sèche sous pression réduite à 90°C. On isole finalement 2,7 g de fumarate. Point de fusion : 149-151°C.

Exemple 8 (Composé N°27)

[[[(Trifluorométhyl-3 phényl)carbonyl]-1 pipéridinyl-4]méthyl]-2 tétrahydro-1,2,3,4 isoquinoléine, fumarate.

A une solution de 4,7 g (0,0226 mole) de chlorure de trifluorométhyl-3 benzoyle dans 20 ml de dichlorométhane, refroidie par un bain de glace, on ajoute, goutte à goutte, un mélange de 5,2 g (0,026 mole) de [(pipéridinyl-4]méthyl]-2 tétrahydro-1,2,3,4 isoquinoléine et 3,8 ml (0,027 mole) de triéthylamine dissoute dans 20 ml de dichlorométhane.

On agite le mélange pendant 20h à température ambiante puis on le filtre, on lave le filtrat à l'eau, on le sèche et on l'évapore sous pression réduite. On purifie l'huile résiduelle par chromatographie sur colonne de silice et on obtient 7 g d'une huile qu'on dissout dans 20 ml de méthanol, on ajoute cette solution à 2,02 g d'acide fumarique en solution dans 20 ml de méthanol, on évapore le mélange, on fait cristalliser le résidu dans de l'éther puis on recristallise le solide dans l'alcool isopropylique. On isole finalement 7,4 g de fumarate. Point de

fusion : 162-164° C.

Exemple 9 (Composé N°41)

[(Phénylcarbonylméthyl-1 pipéridinyl-4)méthyl]-2 tétrahydro1,2,3,4 isoquinoléine, difumarate.

On dissout 3,46 g (15 mmoles) de (pipéridinyl-4 méthyl)-2 tétrahydro-1,2,3,4 isoquinoléine dans 20 ml de dichlorométhane sec, on ajoute 4,05 g (44 mmoles) de triéthylamine sèche puis, à ce mélange, on ajoute goutte à goutte une solution de 3 g (15 mmoles) de bromo-2 phényl-1 éthanone-1 dissoute dans 80 ml de dichlorométhane. On agite la suspension pendant 3h à température ambiante, puis on la concentre sous pression réduite. On lave le résidu d'évaporation à l'eau, on extrait l'huile avec du dichlorométhane, on sépare la phase organique, on la sèche sur sulfate de magnésium et on la concentre sous pression réduite. On purifie le résidu par chromatographie sur colonne de silice en éluant avec un mélange 95/5 de dichlorométhane/méthanol. On récupère une huile rouge que l'on dissout dans l'éther. Il se forme un insoluble que l'on élimine par filtration, et on concentre le filtrat. On prépare le difumarate en dissolvant 2,1 g d'acide fumarique dans 50 ml de méthanol et en y ajoutant 3,34 g de base dissoute dans 30 ml d'acétate d'éthyle. Le sel cristallise, on le lave à l'acétate d'éthyle, puis à l'éther et on le sèche sous vide. Point de fusion : 203-205° C.

Exemple 10 (Composé N°45)

[[[(Fluoro-4 phényl)méthyl]-1 pipéridinyl-4]méthyl]-2 tétrahydro-1,2,3,4 isoquinoléine, difumarate.

Sous atmosphère d'argon on dissout 3,6 g (10 mmoles) de [[[(fluoro-4 phényl)carbonyl]-1 pipéridinyl-4]méthyl]-2 tétrahydro-1,2,3,4 isoquinoléine dans 40 ml de tétrahydrofuranne et, en 10 mn, on ajoute, goutte à goutte, 40 ml (40 mmoles) d'une solution molaire de complexe borane/tétrahydrofuranne dans le tétrahydrofuranne.

On agite le mélange pendant 15 mn, on le chauffe au reflux pendant 4h puis on le laisse reposer à température ambiante.

On détruit le complexe en ajoutant 10 ml de méthanol puis 5 ml d'acide chlorhydrique concentré, et on chauffe le mélange encore pendant 6h au reflux.

On évapore les solvants sous pression réduite, on reprend le résidu avec de l'eau et on en ajuste le pH à 10 au moyen d'ammoniaque concentrée.

On extrait le mélange au moyen d'acétate d'éthyle, on lave la phase organique avec une solution saturée de chlorure de sodium, jusqu'à neutralité, et on la sèche sur sulfate de sodium.

On évapore le solvant sous pression réduite et on obtient 4,2 g d'une huile orange qui cristallise partiellement. On la dissout dans 100 ml d'éthanol, on filtre la solution, et on ajoute au filtrat une solution de 1,85 g (16 mmoles) d'acide fumarique dans 100 ml d'éthanol.

On obtient une solution jaune qui cristallise partiellement. On l'évapore sous pression réduite et on recristallise le résidu dans 150 ml d'éthanol, on le lave à l'éther glacé et on le sèche sous vide. On isole 3,4 g de difumarate. Point de fusion : 183-185° C.

Exemple 11 (Composé N°49)

[[[(Diméthoxy-3,4 phényl)méthyl]-1 pipéridinyl-4]méthyl]-2 tétrahydro-1,2,3,4 isoquinoléine, difumarate.

a) Chlorure de [(diméthoxy-3,4 phényl)méthyl]-1 [(tétrahydro1,2,3,4 isoquinoléinyl-2)carbonyl]-4 pyridinium.

On chauffe au reflux pendant 20h un mélange de 4,76 g (20 mmoles) de [(pyridinyl-4)carbonyl]-2 tétrahydro-1,2,3,4 isoquinoléine, 4,5 g (24 mmoles) de chlorure de diméthoxy-3,4 benzyle et 200 ml d'acétate d'éthyle. On laisse refroidir le mélange obtenu, on sépare le précipité par filtration, et on le lave à l'éther. On recueille 6,6 g de sel qu'on utilise tel quel dans l'étape suivante.

b) [[[(Diméthoxy-3,4 phényl)méthyl]-1 pipéridinyl-4]carbonyl]-2 tétrahydro-1,2,3,4 isoquinoléine.

On introduit dans un flacon de Parr 6,5 g de chlorure de [(diméthoxy-3,4 phényl)méthyl],-1 [(tétrahydro-1,2,3,4 isoquinoléinyl-2)carbonyl]-4 pyridinium, 300 ml d'éthanol et 0,7 g d'oxyde de platine.

On agite le mélange sous une pression de 0,35 MPa d'hydrogène jusqu'à la fin de l'absorption, soit environ 18h. On filtre le catalyseur, on le lave à l'éthanol, on évapore le filtrat, on le neutralise avec de l'ammoniaque et on l'extrait avec du dichlorométhane.

On lave la phase organique à l'eau, on la sèche et on évapore le solvant. On recueille 6 g d'une huile qu'on utilise telle quelle dans l'étape suivante.

c) [[[(Diméthoxy-3,4 phényl)méthyl]-1 pipéridinyl-4]méthyl]-2 tétrahydro-1,2,3,4 isoquinoléine.

On agite pendant 10 mn une suspension de 0,6 g (15,8 mmoles) d'hydrure de lithium et d'aluminium dans 50 ml de tétrahydrofuranne, puis on ajoute lentement 3 g (7,6 mmoles) de [[[(diméthoxy-3,4 phényl)méthyl]-1 pipéridinyl-4]carbonyl]-2 tétrahydro-1,2,3,4 isoquinoléine en solution dans 100 ml de tétrahydrofuranne.

On chauffe le mélange au reflux pendant 5h, on le laisse refroidir, on ajoute de la soude 1N, on agite pendant 20 mn, on filtre le précipité minéral, en le lavant à l'éther, on lave le filtrat à l'eau, on le sèche et on l'évapore. On recueille 2,2 g d'une huile jaune dont on prépare le difumarate en la traitant avec deux équivalents d'acide fumarique, et on recristallise le difumarate dans l'éthanol. Point de fusion : 175-176°C.

Exemple 12 (Composé N°51)

[[[(Cyano-3 phényl)méthyl]-1 pipéridinyl-4]méthyl]-2 tétrahydro-1,2,3,4 isoquinoléine, difumarate.

On agite pendant 15h un mélange de 6,79 g (31 mmoles) de [(pipéridinyl-4)méthyl]-2 tétrahydro-1,2,3,4 isoquinoléine, 7,29 g (37 mmoles) de bromométhyl-3 benzénenitrile, 10,7 g (77 mmoles) de carbonate de potassium et 70 ml d'acétone. On filtre le mélange, on évapore le filtrat, on ajoute de l'eau ét du dichlorométhane, on sépare la phase organique, on la lave à l'eau, on la sèche sur sulfate de magnésium et on l'évapore. On purifie le résidu par chromatographie sur colonne de silice en éluant d'abord avec un mélange 97/3 de dichlorométhane/méthanol, puis 96/4 de dichlorométhane/méthanol. On obtient 6,2 g d'une huile jaune.

On en dissout 3 g (8,6 mmoles) dans de l'acétate d'éthyle, et on verse cette solution dans une solution de 1,99 g (17,2 mmoles) d'acide fumarique dissout dans le minimum d'éthanol. On recueille finalement 4,1 g de difumarate. Point de fusion : 179-181°C.

Exemple 13 (Composé N°52)

[[[(Aminocarbonyl-3 phényl)méthyl]-1 pipéridinyl-4]méthyl]-2 tétrahydro-1,2,3,4 isoquinoléine, difumarate.

On fait barboter un courant d'acide chlorhydrique gazeux à travers un mélange de 2,3 g (66 mmoles) de [[[(cyano-3 phényl)méthyl]-1 pipéridinyl-4]méthyl]-2 tétrahydro-1,2,3,4 isoquinoléine base libre) et 30 ml d'acide formique, en suivant la disparition du composé de départ par chromatographie sur couche mince.

Lorsque la réaction est terminée on ajoute de l'eau, de l'ammoniaque et du dichlorométhane, on sépare la phase organique, on la lave à l'eau, on la sèche sur sulfate de magnésium, on la filtre et on l'évapore. On obtient 3,18 g d'une huile jaune qu'on purifie par chromatographie sur colonne de silice en éluant avec des mélanges de dichlorométhane/méthanol dans des proportions successives de 95/5, 94/6, 93/7, 92/8, 91/9 et 90/10. On isole ainsi 1,5 g (4,1 mmoles) de base pure qu'on dissout dans de l'acétate d'éthyle et qu'on verse goutte à goutte dans 0,85 g (8,2 mmoles) d'acide fumarique en solution dans le minimum de méthanol. On évapore le mélange obtenu, et on le lave à l'éther puis à l'acétate d'éthyle. On isole finalement 1,5 g de difumarate. Point de fusion : 182-184°C.

Exemple 14 (Composé N°53)

[[[(Nitro-3 phényl)méthyl]-1 pipéridinyl-4]méthyl]-2 tétrahydro-1,2,3,4 isoquinoléine difumarate.

a) [[[(Nitro-3 phényl)méthyl]-1 pipéridinyl-4]carbonyl]-2 tétrahydro-1,2,3,4 isoquinoléine.

On agite pendant 15h un mélange de 7 g (28,6 mmoles) de [(pipéridinyl-4)carbonyl]-2 tétrahydro-1,2,3,4 isoquinoléine, 6,8 g (31,5 mmoles) de α-bromo nitro-3 toluène, et 6 g (43 mmoles) de carbonate de potassium dans 140 ml d'acétone. On évapore le solvant, on ajoute de l'eau et du dichlorométhane, on sépare la phase organique, on la neutralise, on la sèche et on l'évapore. On obtient 13 g d'une huile jaune qu'on purifie par chromatographie sur colonne de silice en éluant avec un mélange 98/2 de dichlorométhane/méthanol. Après recristallisation dans l'éther on obtient 9,6 g de produit qu'on utilise tel quel dans l'étape suivante.

b) [[[(Nitro-3 phényl)méthyl]-1 pipéridinyl-4]méthyl]-2 tétrahydro-1,2,3,4 isoquinoléine, difumarate.

On chauffe au reflux pendant 5h un mélange de 6,6 g (17,4 mmoles) de [[[(nitro-3 phényl)méthyl]-1 pipéridinyl-4]carbonyl]-2 tétrahydro-1,2,3,4 isoquinoléine et 1,44 g (52 mmoles) de diborane dans 90 ml de tétrahydrofuranne. On laisse reposer le mélange, puis on l'hydrolyse en ajoutant 20 ml de méthanol et 5 ml d'acide chlorhydrique concentré. On le chauffe au reflux pendant 3h, on l'évapore, on le reprend à l'eau, on

ajoute du charbon actif, on l'agite pendant 1h à température ambiante, on le filtre et on neutralise le filtrat. On en sépare la phase organique, on la sèche, on évapore le solvant sous pression réduite et on purifie le résidu par chromatographie sur colonne de silice en éluant avec un mélange 97/3 de dichlorométhane/ méthanol. Après recristallisation dans le pentane on obtient 5,27 g de base purifiée. On prépare la difumarate en traitant 2,5 g (6,84 mmoles) de base avec 1,59 g (13,7 mmoles) d'acide fumarique, dans le méthanol. Point de fusion : 216-217°C.

Exemple 15 (Composé N°54)

[[[(Amino-3 phényl)méthyl]-1 pipéridinyl-4]méthyl]-2 tétrahydro-1,2,3,4 isoquinoléine, difumarate.

On soumet une solution de 2,7 g (7,4 mmoles) de [[[(nitro-3 phényl)méthyl]-1 pipéridinyl-4]méthyl]-2 tétrahydro-1,2,3,4 isoquinoléine, dans 135 ml d'acide acétique à une hydrogénation catalytique sous une pression de 0,35 MPa, en présence de charbon palladié, jusqu'à la fin de l'absorption d'hydrogène.

On filtre le catalyseur, on concentre le filtrat sous pression réduite, et on purifie le résidu par chromatographie sur colonne de silice en éluant avec un mélange 90/10 de dichlorométhane/méthanol. Après recristallisation dans le pentane on recueille 1,9 g de base purifiée.

On dissout 0,242 g (2,08 mmoles) d'acide fumarique et 0,349 g (1,04 mmoles) de base dans le minimum d'éthanol, on réunit les deux solutions, on évapore le solvant et on recristallise le résidu dans l'alcool isopropylique. On isole finalement 0,460 g de difumarate. Point de fusion : 163-166°C.

Exemple 16 (Composé N°55)

[[[Diméthylamino-3 phényl)méthyl]-1 pipéridinyl-4]méthyl]-2 tétrahydro-1,2,3,4 isoquinoléine, difumarate.

On dissout 0,805 g (26,8 mmoles) de formaldéhyde dans 30 ml de tétrahydrofuranne et on ajoute 1,1 g (11,2 mmoles) d'acide sulfurique concentré puis 1,5 g (4,47 mmoles) de [[[(amino-3 phényl)méthyl]-1 pipéridinyl-4]méthyl]-2 tétrahydro-1,2,3,4 isoquinoléine. On agite le mélange pendant 30 mn à température ambiante, puis on le refroidit sur un bain de glace et on lui ajoute peu à peu 1,184 g (31,3 mmoles de borohydrure de sodium, sans que le pH devienne supérieur à 4.

On maintient l'agitation à température ambiante pendant 3h, puis on évapore le mélange, on ajoute de l'eau carbonatée et du dichlorométhane au résidu, on sépare la phase organique, on la filtre,,on la sèche, on évapore le solvant sous pression réduite et on purifie le résidu par chromatographie sur colonne de silice. On obtient 0,400 g de base purifiée. On en utilise 0,364 g (1 mmole) pour préparer le difumarate avec 0,221 g (1,9 mmole) d'acide fumarique, selon le mode opératoire décrit à l'exemple précédent. On recueille finalement 0,250 g de difumarate. Point de fusion : 180-182°C.

Exemple 17 (Composé N°61)

[[[(Pyridinyl-2carbonyl]-1 pipéridinyl-4]méthyl]-2 tétrahydro-1,2,3,4 isoquinoléine, fumarate.

Sous atmosphère d'argon on ajoute 10,05 ml (24 mmoles) de triméthylaluminium (à 25 % dans l'hexane) à 20 ml de toluène. On refroidit le mélange par un bain de glace et on ajoute 4,6 g (20 mmoles) de [(pipéridinyl-4)méthyl]-2 tétrahydro1,2,3,4 isoquinoléine dilués dans 25 ml de toluène.

On chauffe le mélange à environ 50° C et on ajoute 3,02 g (20 mmoles) de pyridine-2-carboxylate d'éthyle en solution dans 15 ml de toluène, on élimine environ 10 ml de solvants à l'aide d'un appareil de Dean-Stark, et on chauffe le mélange au reflux pendant 3h.

Ensuite on le refroidit par un bain de glace, on l'hydrolyse avec 10 ml d'eau, on le filtre en rinçant le solide avec de l'acétate d'éthyle, on lave le filtrat trois fois à l'eau, on le sèche sur sulfate de magnésium, on le filtre et on évapore le solvant sous pression réduite.

On obtient 6,7 g (20 mmoles) de base libre, qu'on dissout dans le minimum d'éthanol, on ajoute une solution de 2,3 g (20 mmoles) d'acide fumarique dans 300 ml d'éthanol. On évapore l'éthanol et on recristallise le résidu dans l'éthanol. On isole finalement 5 g de fumarate. Point de fusion : 115-118°C

Exemple 18 (Composé N°68)

[[[(Indolyl-2)carbonyl]-1 pipéridinyl-4]méthyl]-2 tétrahydro1,2,3,4 isoquinoléine, fumarate.

Sous atmosphère d'argon on ajoute, par petites portions, 4,05 g (25 mmoles) de N,N'-carbonyldiimidazole à une solution de 3,7 g (23 mmoles) d'acide indole-2-carboxylique dans 37 ml de tétrahhydrofuranne.

On agite le mélange pendant 3h à température ambiante, puis on évapore le solvant. On reprend le résidu

avec 46 ml de dichlorométhane et on ajoute cette solution à une solution de 4,6 g (20 mmoles) de [pipéridinyl-4)méthyl]-2 tétrahydro 1,2,3,4 isoquinoléine dans 40 ml de dichlorométhane. On agite le mélange pendant 12h, puis on le verse dans de l'eau. On l'extrait avec du dichlorométhane, on lave la phase organique quatre fois à l'eau, on la sèche sur sulfate de magnésium, on la filtre, on évapore le solvant sous pression réduite et on recristallise le résidu dans du tétrahydrofuranne.

On obtient 5 g (13,3 mmoles) de base libre qu'on introduit dans un mélange de dichlorométhane et de méthanol, et on ajoute une solution de 1,55 g (13,3 mmoles) d'acide fumarique dans 200 ml d'éthanol. On chauffe le mélange au reflux, on ajoute de l'éthanol jusqu'à dissolution complète, puis on évapore les solvants sous pression réduite et on recristallise le résidu dans l'éthanol. On isole finalement 5,5 g de fumarate. Point de fusion : 203-206°C

Exemple 19 (Composé N°65)

[[[(Thiényl-3)carbonyl]-1 pipéridinyl-4]méthyl]-2 tétrahydro1,2,3,4 isoquinoléine, fumarate,

a) Chlorure d'acide thiophéne-3-carboxylique.

On ajoute 5,12 g (44 mmoles) d'acide thiophène-3-carboxylique à 50 ml de chlorure de thionyle, on chauffe le mélange à l'ébullition pendant 2h, puis on l'évapore, on reprend le résidu avec du toluène, et on évapore ce dernier. On obtient 5 g de résidu qu'on utilise tel quel dans l'étape suivante.

b) [[[(Thiényl-3)carbonyl]-1 pipéridinyl-4]méthyl]-2 tétrahydro-1,2,3,4 isoquinoléine.

A une solution de 4,6 g (20 mmoles) de [(pipéridinyl-4)méthyl]-2 tétrahydro-1,2,3,4 isoquinoléine et 3,06 ml (22 mmoles) de triéthylamine dans 46 ml de dichlorométhane on ajoute, à température ambiante, 2,5 g (17 mmoles) de chlorure d'acide thiophène-3-carboxylique en solution dans 20 ml de dichlorométhane, et on agite le mélange pendant 48h. La réaction n'étant pas terminée, on ajoute encore 2,5 g (17 mmoles) de chlorure d'acide et 3 ml (22 mmoles) de triéthylamine en solution dans 20 ml de dichlorométhane.

On agite le mélange encore pendant lh, on le verse dans l'eau, on l'extrait avec du dichlorométhane, on lave la phase organique deux fois à l'eau, on la sèche sur sulfate de magnésium, on la filtre et on évapore le solvant sous pression réduite.

On purifie le résidu par chromatographie sur colonne de silice en éluant avec un mélange 98/2 de dichlorométhane/méthanol. On obtient 3 g (8,8 mmoles) de base, que l'on dissout dans un mélange de dichlorométhane et d'éthanol, on ajoute une solution de 1 g (8,8 mmoles) d'acide fumarique dans 100 ml d'éthanol. On évapore les solvants et on recristallise le résidu dans l'éthanol. On isole finalement 3,3 g de fumarate. Point de fusion : 174-177°C

Exemple 20 (Composé N°74)

[[(Trifluoroacétyl)-1 pipéridinyl-4]méthyl]-2 tétrahydro1,2,3,4 isoquinoléine, chlorhydrate.

A un mélange de 2,3 g (10 mmoles) de [(pipéridinyl-4)méthyl]-2 tétrahydro-1,2,3,4 isoquinoléine, 1,4 ml (10 mmoles) de triéthylamine et 100 ml de tétrahydrofuranne, refroidi à 0°C, on ajoute goutte à goutte 2,3 g (11 mmoles) d'anhydride trifluoroacétique en solution dans 50 ml de tétrahydrofuranne. On laisse revenir la température à 20°C et on maintient l'agitation pendant 20h.

On filtre le mélange, on lave le filtrat avec une solution aqueuse de soude à 10 %, puis avec de l'eau, on le sèche sur sulfate de magnésium, on le filtre et on évapore le solvant sous pression réduite.

On obtient 1,7 g de résidu huileux qui cristallise. On en prépare la chlorhydrate par addition d'alcool isopropylique chlorhydrique O,1N et on le recristallise dans l'éthanol. Pôint de fusion : 195-196°C.

Exemple 21 (Composé N°19)

[[[(Méthyl-3 phényl)carbonyl]-1 pipéridinyl-4]méthyl]-2 tétrahydro-1,2,3,4 isoquinoléine, fumarate.

a) Pipéridine-4-méthanol.

Dans un bailon tricol de 4 l muni d'un système d'agitation mécanique et d'un réfrigérant on introduit 28,5 g (0,75 mole) d'hydrure de lithium et aluminium et 1,2 l de tétrahydrofuranne. On ajoute à la suspension obtenue 117,9 g (0,75 mole) de pipéridine-4-carboxylate d'éthyle en solution dans 1,2 l de tétrahydrofuranne, et on agite le mélange pendant 6h à 20°C. On le refroidit à 0°C, puis on l'hydrolyse en ajoutant successivement 22 ml

d'eau, 22 ml d'hydroxyde de sodium 1N et 46 ml d'eau. On agite le mélange 30mn à 20°C, on le filtre, on lave le précipité au tétrahydrofuranne puis à l'éther. On évapore les solvants sous pression réduite, et on obtient 84,4 g d'une huile qu'on utilise telle quelle dans l'étape suivante.

b) Méthyl-3 benzoate de [(méthyl-3 benzoyl)-1 pipéridinyl-4]méthyle.

Dans un ballon tricol de 3 l on introduit, sous atmosphère d'argon, 42,25 g (0,367 mole) de pipéridine-4-méthanol, 430 ml de dichloro-1,2 éthane, et on ajoute 82 g (0,81 mole) de triéthylamine puis 125,2 g (0,81 mole) de chlorure de méthyl-3 benzoyle. On chauffe le mélange au reflux pendant 4h30, on ajoute encore 8,2 g (0,08 mole) de triéthylamine et 12,5 g (0,08 mole) de chlorure de méthyl-3 benzoyle, et on chauffe le mélange pendant encore 3h.

On le filtre, on lave les sels au dichloro-1,2 éthane, on évapore le filtrat sous pression réduite, on dissout le résidu dans de l'acétate d'éthyle, on lave la solution avec une solution aqueuse saturée de chlorure de sodium, on évapore le solvant sous pression réduite, et on recristallise le résidu dans un mélange 1/1 d'alcool isopropylique/acétate d'éthyle. On obtient 80 g d'un solide blanc. Point de fusion : 80-83°C.

c) (Méthyl-3 benzoyl)-1 pipéridine-4-méthanol.

A une solution de 80 g (0,23 mole) de méthyl-3 benzoate de [(méthyl-3 benzoyl)-1 pipéridinyl-4]méthyle dans 400 ml d'éthanol, on ajoute une solution de 12,76 g (0,23 mole) d'hydroxyde de potassium dans 75 ml d'éthanol et 75 ml d'eau. On agite le mélange à 20°C pendant 3h, on évapore le solvant sous pression réduite et on extrait la phase aqueuse à l'acétate d'éthyle. On lave la phase organique avec de l'eau puis avec une solution aqueuse saturée de chlorure de sodium, et on la sèche sur sulfate de magnésium. On évapore le solvant sous pression réduite et on obtient 53 g d'alcool qu'on utilise tel quel dans l'étape suivante.

d) Méthyl-4 benzènesulfonate de [(méthyl-3 benzoyl)-1 pipéridinyl-4]méthyle.

A une solution de 52 g (0,22 mole) de (méthyl-3 benzoyl)-1 pipéridine-4-méthanol dans 100 ml de pyridine on ajoute 53,3 g (0,28 mole) de chlorure de méthyl-4 benzènesulfonyle dans 60 ml de pyridine. On agite le mélange à 20°C pendant 4h, puis on le verse dans de la glace. On extrait la phase au dichlorométhane, on lave la phase organique avec une solution aqueuse 10N d'acide chlorhydrique et on la sèche sur sulfate de magnésium. On évapore les solvants sous pression réduite et on obtient 70 g de solide blanc. Point de fusion : 68-70°C.

e) [[[(Méthyl-3 phényl)carbonyl]-1 pipéridinyl-4]méthyl]-2 tétrahydro-1,2,3,4 isoquinoléine, fumarate.

On chauffe pendant 4h à 150°C un mélange de 2,66 g (0,02 mole) de tétrahydro-1,2,3,4 isoquinoléine et 7,75 g (0,02 mole) de méthyl-4 benzènesulfonate de [(méthyl-3 benzoyl)-1 pipéridinyl-4]méthyle. On laisse refroidir le mélange, on le dissout dans du dichlorométhane et on ajoute de l'ammoniaque concentrée. On sépare la phase organique, on la lave trois fois à l'eau, on la sèche sur sulfate de magnésium, on l'évapore sous pression réduite et on purifie l'huile résiduelle par chromatographie sur colonne de silice en éluant avec un mélange 98/2 de dichlorométhane/méthanol. On obtient 2 g (0,0057 mole) de base pure qu'on dissout dans de l'éthanol, on ajoute 0,66g (0,0057 mole) d'acide fumarique en solution dans 100 ml d'éthanol, on évapore l'éthanol et on recristallise le résidu dans l'acétone. On isole finalement 2 g de fumarate. Point de fusion : 149-151°C.

Exemple 22 (Composé N°8)

[[[(Méthyl-3 phényl)méthyl]-1 pipéridinyl-4]méthyl]-2 tétrahydro-1,2,3,4 isoquinoléine, difumarate.

On dissout 1,36 g de [[[(méthyl-3 phényl)carbonyl]-1 pipéridinyl-4]méthyl]-2 tétrahydro-1,2,3,4 isoquinoléine dans 30 ml d'éther sec, on ajoute en une fois 0,29 g d'hydrure de lithium et aluminium et on agite le mélange à température ambiante pendant 3h. On le refroidit par un bain de glace, on l'hydrolyse avec 2,4 ml d'hydroxyde de sodium 1N, on le filtre et on évapore le filtrat. On obtient 1,2 g (0,0035 mole) de base qu'on dissout dans le minimum d'éthanol, on ajoute 0,82 g (0,0035 moles) d'acide fumarique dissous dans 100 ml d'éthanol, on filtre le précipité et on le sèche. On obtient finalement 1,2 g de difumarate. Point de fusion : 185-186°C.

Le tableau suivant illustre les structures chimiques et les propriétés physiques de quelques composés selon l'invention.

Tableau

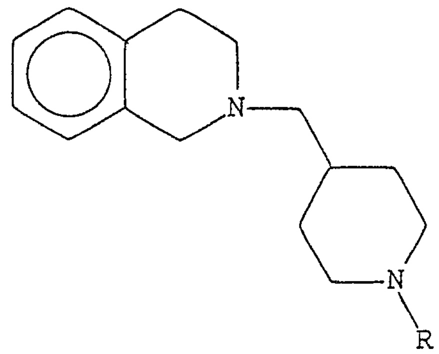

(I)

| N° | R | Sel | F(°C) |
|---|---|---|---|
| 1 | H | diHCl | 252-255 |
| 2 | $CH_3$ | difum. | 157-158 |
| 3 | $C_6H_5-CH_2-$ | diHCl | 259-264 |
| 4 | $2-Cl-C_6H_4-CH_2-$ | difum. | 209-211 |
| 5 | $3-Cl-C_6H_4-CH_2-$ | difum. | 188-190 |
| 6 | $4-Cl-C_6H_4-CH_2-$ | difum. | 205-207 |
| 7 | $2-CH_3-C_6H_4-CH_2-$ | difum. | 182-183 |
| 8 | $3-CH_3-C_6H_4-CH_2-$ | difum. | 185-186 |
| 9 | $4-CH_3-C_6H_4-CH_2-$ | difum. | 190,5-193 |
| 10 | $2-CH_3O-C_6H_4-CH_2-$ | difum. | 177-180 |
| 11 | $3-CH_3O-C_6H_4-CH_2-$ | difum. | 173-174 |
| 12 | $4-CH_3O-C_6H_4-CH_2-$ | difum. | 204-206,5 |
| 13 | $C_6H_5-CH_2-CH_2-$ | difum. | 212-213 |
| 14 | $C_6H_5-CO-$ | HCl | 228-235 |
| 15 | $2-Cl-C_6H_4-CO-$ | fum. | 95-98 |
| 16 | $3-Cl-C_6H_4-CO-$ | HCl | 162-163 |
| 17 | $4-Cl-C_6H_4-CO-$ | fum. | 168-170 |
| 18 | $2-CH_3-C_6H_4-CO-$ | fum. (1) | 115-120 |
| 19 | $3-CH_3-C_6H_4-CO-$ | fum. | 149-151 |
| 20 | $4-CH_3-C_6H_4-CO-$ | fum. | 134-136 |
| 21 | $2-OCH_3-C_6H_4-CO-$ | fum. | 110-111 |
| 22 | $3-OCH_3-C_6H_4-CO-$ | fum. | 127-128 |
| 23 | $4-OCH_3-C_6H_4-CO-$ | fum. | 132-133 |
| 24 | $2-F-C_6H_4-CO-$ | fum. | 131-133 |

Tableau (suite)

| N° | R | Sel | F(°C) |
|---|---|---|---|
| 25 | $3-F-C_6H_4-CO-$ | fum. | 166-167 |
| 26 | $4-F-C_6H_4-CO-$ | fum. | 172-174 |
| 27 | $3-CF_3-C_6H_4-CO-$ | fum. | 162-164 |
| 28 | $4-CF_3-C_6H_4-CO-$ | fum. | 194-195 |
| 29 | $4-NO_2-C_6H_4-CO-$ | HCl | 209-210 |
| 30 | $4-F-C_6H_4-CO-$ | fum. | 172-174 |
| 31 | $4-SCH_3-C_6H_4-CO-$ | fum. | 164-166 |
| 32 | $3,5-Cl_2-C_6H_3-CO-$ | fum. | 165-166 |
| 33 | $2,3-(OCH_3)_2-C_6H_3-CO-$ | fum. | 185-186 |
| 34 | $3,5-(OCH_3)_2-C_6H_3-CO-$ | fum.(2) | 83-86 |
| 35 | $3,5-(CH_3)_2-C_6H_3-CO-$ | fum. | 90-95 |
| 36 | $3,5-(CF_3)_2-C_6H_3-CO-$ | fum.(3) | 105-108 |
| 37 | $C_{10}H_7-\alpha-CO-$ * | fum. | 103-107 |
| 38 | $C_{10}H_7-\beta-CO-$ * | fum. | 163-165 |
| 39 | $CH_3-CH_2-CH_2-$ | difum. | 186-187 |
| 40 | $CH_2=CH-CH_2-$ | difum. | >250 |
| 41 | $C_6H_5-CO-CH_2-$ | difum. | 203-205 |
| 42 | $C_6H_5-CH_2-CH_2-CH_2-$ | difum. | 153-154 |
| 43 | $2-F-C_6H_4-CH_2-$ | difum. | 189-191 |
| 44 | $3-F-C_6H_4-CH_2-$ | difum. | 184-186 |
| 45 | $4-F-C_6H_4-CH_2-$ | difum. | 183-185 |
| 46 | $3,5-F_2-C_6H_3-CH_2-$ | difum. | 188-190 |
| 47 | $3,5-Cl_2-C_6H_3-CH_2-$ | difum. | 198-199 |
| 48 | $3,5-(CH_3)_2-C_6H_3-CH_2-$ | difum. | 203-205 |
| 49 | $3,4-(CH_3O)_2-C_6H_3-CH_2-$ | difum. | 175-176 |
| 50 | $3,5-(CH_3O)_2-C_6H_3-CH_2-$ | difum. | 199-201 |
| 51 | $3-NC-C_6H_4-CH_2-$ | difum. | 179-181 |
| 52 | $3-H_2NCO-C_6H_4-CH_2-$ | difum. | 182-184 |
| 53 | $3-O_2N-C_6H_4-CH_2-$ | difum. | 216-217 |
| 54 | $3-H_2N-C_6H_4-CH_2-$ | difum. | 163-166 |
| 55 | $3-(CH_3)_2N-C_6H_4-CH_2-$ | difum. | 180-182 |
| 56 | $4-CH_3S-C_6H_4-CH_2-$ | difum. | 196-198 |
| 57 | $3-CF_3-C_6H_4-CH_2-$ | difum. | 193-195 |
| 58 | $3,5-(CF_3)_2-C_6H_3-CH_2-$ | difum. | 222-224 |

Tableau (suite)

| N° | R | Sel | F(°C) |
|---|---|---|---|
| 59 | $3-C_2H_5O-C_6H_4-CH_2-$ | difum. | 190-192 |
| 60 | $C_6H_5-CH=CH-CH_2-$ (trans) | diHCl | 197-198 |
| 61 | 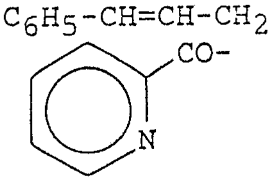 | fum. | 115-118 |
| 62 | 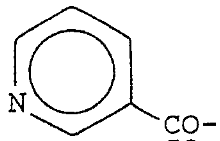 | fum. | 180-182 |
| 63 | 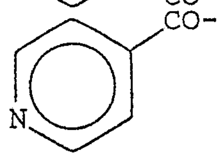 | fum. | 179-180 |
| 64 | 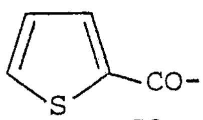 | fum. | 169-172 |
| 65 | 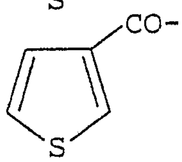 | fum. | 174-177 |
| 66 | 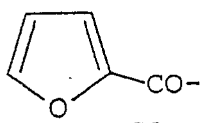 | fum. | 151-152 |
| 67 | 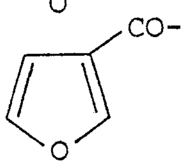 | fum. | 171-174 |
| 68 | 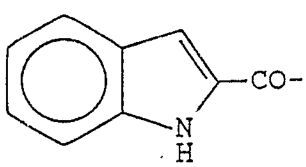 | fum. | 203-206 |
| 69 | 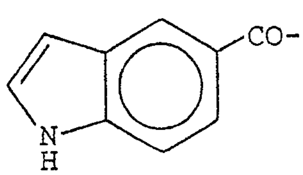 | fum.(2) | 110-125 |
| 70 | $CH_3-CO-$ | fum. | 139-141 |
| 71 | $nC_3H_7-CO-$ | fum. | 168-170 |
| 72 | $cC_6H_{11}-CO-$ | fum. | 202-204 |

Tableau (fin)

| N° | R | Sel | F(°C) |
|---|---|---|---|
| 73 | $C_6H_5-CH_2-CO-$ | fum. | 132-136 |
| 74 | $CF_3-CO-$ | HCl | 195-196 |
| 75 | $C_6H_5-CH=CH-CO-$ (trans) | HCl | 214-215 |
| 76 | $C_{10}H_7-\alpha-CH_2-$ * | difum. | 208-212 |
| 77 | $C_{10}H_7-\beta-CH_2-$ * | difum. | 190-192 |
| 78 | 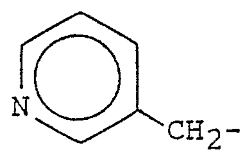 | difum. | 192-193 |
| 79 | 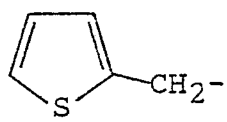 | difum. | 191-193 |
| 80 | $3,5-F_2-C_6H_3-CO-$ | fum. | 177-179 |
| 81 | $3-C_2H_5O-C_6H_4-CO-$ | fum. | 74-77 |

<u>Notes</u> :

fum. : fumarate

difum. : difumarate

HCl : chlorhydrate

diHCl : dichlorhydrate

1) fumarate contenant un équivalent d'alcool isopropylique ;

2) fumarate hémihydraté ;

3) fumarate monohydraté ;

*) $C_{10}H_7-\alpha$ et $C_{10}H_7-\beta$ représentent les groupes α- et β-naphtyle, respectivement.

Les composés de l'invention ont été soumis a une série d'essais pharmacologiques qui ont mis en évidence leur intérêt comme substances à activités thérapeutiques.

Ainsi ils ont fait l'objet d'une étude quant à leur affinité pour les récepteurs sérotoninergiques du type $5\text{-}HT_{1A}$ présents dans l'hippocampe du rat.

Les composés déplacent la liaison d'un ligand spécifique marqué, la [$^3$H]-hydroxy-8 di-n-propylamino-2 tétraline (désignée ci-après par "[$^3$H]-8-OH-DPAT" et décrite par Gozlan et coll., Nature, (1983), 305, 140-142) sur les récepteurs $5\text{-}HT_{1A}$.

Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés trois fois à 4° C, en les centrifugeant à chaque fois pendant 10 mn à 48000xg et en remettant le culot en suspension dans du tampon frais refroidi. Finalement on met le dernier culot en suspension dans le tampon pour arriver à une concentration de 100 mg de tissu de départ par ml de tampon à 50 mM. On laisse ensuite incuber à 37° C pendant 10 mn. La liaison avec la [$^3$H]-8-OH-DPAT (1 nM) est déterminée par incubation de 100 µl de suspension de membranes dans un volume final de 1 ml de tampon contenant 10 µM de pargyline et 3 µM de paroxétine.

Après une incubation de 15 mn à 37° C on récupère les membranes par filtration sur filtres Whatman GF/B qu'on lave trois fois avec.des, quantités aliquotes de 5 ml de tampon glacé. On extrait les filtres dans le liquide de scintillation et on en mesure la radioactivité par scintigraphie liquide. La liaison spécifique de la [$^3$H]-8-OH-DPAT est définie comme la quantité radioactive retenue sur les filtres et pouvant être inhibée par co-incubation dans la hydroxy-5 tryptamine à 10 µM. A une concentration de 1 nM de [$^3$H]-8-OH-DPAT la liaison spécifique représente 90% de la radioactivité totale recupérée sur le filtre.

Pour chaque concentration de composés étudié on détermine le pourcentage d'inhibition de la liaison avec la [3H]-8-OHDPAT, puis la concentration $CI_{50}$, concentration qui inhibe 50% de la liaison.

Pour les composés de l'invention les $CI_{50}$ se situent entre 0,001 et 0,3 µM.

L'activité centrale des composés de l'invention a été évaluée par leurs effets sur les "pointes PGO" (ponto-géniculo-occipitales) induites par la réserpine test PGO-R) chez le chat, selon la méthode décrite par H. Depoortere, Sleep 1976, 3rd Europ. Congr. Sleep Res., Montpellier 1976, 358-361 (Karger, Basel 1977).

On administre des doses cumulatives de composés à étudier (de 0,001 à 3 mg/kg par voie intraveineuse) à des intervalles de temps de 30 mn, 4h après injection intrapéritonéale d'une dose de 0,75 mg/kg de réserpine, à des chats curarisés, sous ventilation artificielle. On recueille les activités électroencéphalographique et phasique (pointes PGO-R) à l'aide d'électrodes corticales et profondes (genouillé latéral). Pour chaque dose de composé étudié on détermine le pourcentage de diminution du nombre de pointes PGO, puis la $DA_{50}$, dose active qui diminue de 50% ce nombre de pointes. Pour les composés de l'invention les $DE_{50}$ se situent entre 0,001 et 3 mg/kg par la voie intraveineuse.

Les résultats des essais montrent que les composés de formule générale (I) possèdent, in vitro, une grande affinité et une sélectivité pour les récepteurs sérotoninergiques de type $5\text{-}HT_{1A}$. In vivo ils montrent une activité agoniste, agoniste partielle, ou antagoniste au niveau de ces récepteurs.

Les composés de l'invention peuvent donc être utilisés pour le traitement des maladies et affections impliquant les récepteurs sérotoninergiques de type $5\text{-}HT_{1A}$ de façon directe ou indirecte, notamment pour le traitement des états dépressifs, des états d'anxiété, des troubles du sommeil, et pour la régulation de la prise de nourriture, et également pour le traitement de désordres vasculaires, cérébrovasculaires ou cardiovasculaires tels que la migraine et l'hypertension.

A cet effet ils peuvent être présentés sous toutes formes appropriées à leur administration par voie orale ou parentérale, associés à tous excipients convenables, et dosés pour permettre une posologie journalière de 1 à 1000 mg.

**Revendications**

1. Composés répondant à la formule générale (I)

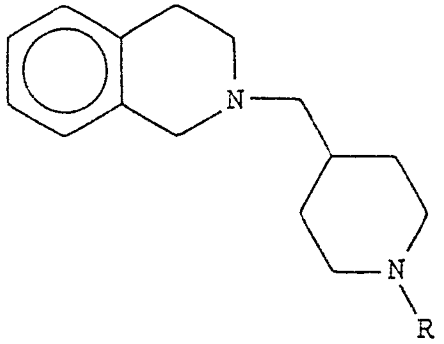

(I)

dans laquelle R représente

a) soit un atome d'hydrogène ;

b) soit un groupe ($C_1$-$C_6$)alkyle linéaire ou ramifié ; un groupe allyle ; un groupe ($C_3$-$C_6$)cycloalkylméthyle ; un groupe phénylméthyle portant éventuellement de un à trois substituants choisis parmi les atomes d'halogènes et les groupes trifluorométhyle, nitro, amino, diméthylamino, cyano, aminocarbonyle, ($C_1$-$C_3$)alkyie linéaires ou ramifiés, ($C_1$-$C_3$)alcoxy linéaires ou ramifiés, ($C_1$-$C_3$)alkylthio linéaires ou ramifiés ; un groupe phényl-2 éthyle ; un groupe phényl-3 propyle ; un groupe phényl-3 propène-2 yle ; un groupe phénylcarbonylméthyle ; un groupe naphtylméthyle ; un groupe pyridinylméthyle ; un groupe furannylméthyle ; ou un groupe thiénylméthyle ;

c) soit un groupe ($C_2$-$C_6$)alcanoyle linéaire ou ramifié ; un groupe ($C_3$-$C_6$)cycloalkylcarbonyle ; un groupe trifluoroacétyle ; un groupe phénylcarbonyle portant éventuellement de un à trois substituants choisis parmi les atomes d'halogènes et les groupes trifluorométhyle, nitro, ($C_1$-$C_3$)alkyle linéaires ou ramifiés, ($C_1$-$C_3$)alcoxy linéaires ou ramifiés, ($C_1$-$C_3$)alkylthio linéaires ou ramifiés ; un groupe oxo-1 phényl-3 propène-2 yle ; un groupe naphtylcarbonyle ; un groupe pyridinylcarbonyle ; un groupe furannylcarbonyle ; un groupe thiénylcarbonyle ; un groupe (indolyl-2)carbonyle ; ou un groupe (indolyl-5)carbonyle,

ainsi que leurs sels d'addition à des acides acceptables en pharmacologie.

2. Procédé de préparation des composés selon la revendication 1, caractérisé en ce qu'on fait réagir la tétrahydro-1,2,3,4 isoquinoléine avec l'imidazolide de formule (III)

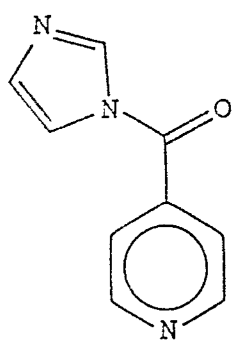

(III)

(préparé in situ au moyen d'acide isonicotinique et de N,N'-carbonyldiimidazole), obtenant ainsi l'amide de formule (IV)

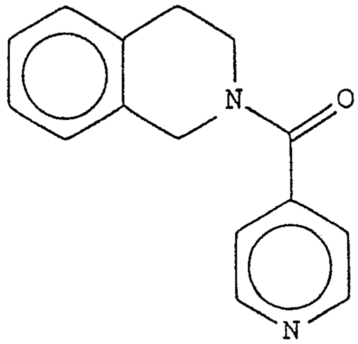

(IV)

que l'on soumet à une hydrogénation catalytique, donnant l'amide de formule (VI)

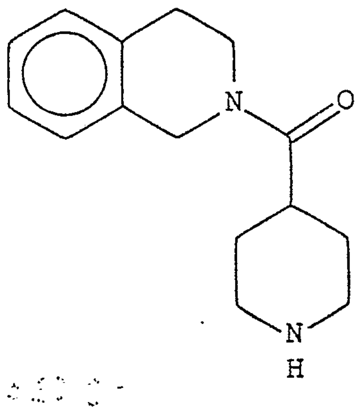

(VI)

qu'on réduit au moyen d'hydrure de lithium et d'aluminium ou d'un hydrure simple ou complexe de bore, tel que le diborane ou le complexe de borane/sulfure de méthyle, obtenant ainsi le composé de formule (IX)

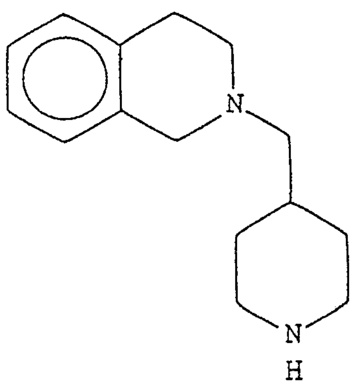

(IX)

qui correspond au composé de formule générale (I) dans laquelle R représente un atome d'hydrogène,
ou bien
on soumet l'amide de formule (VI) à une alkylation au moyen d'un halogénure de formule générale RX dans laquelle R est un groupe "réduit" tel que défini ci-dessus selon b) et X est un groupe labile, par exemple un atome de brome, et finalement on réduit l'amide de formule générale (VII)

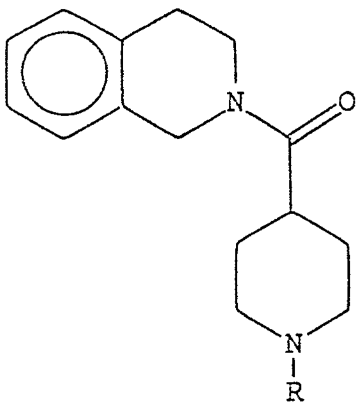

(VII)

ainsi obtenu de la manière décrite à propos de l'amide de formule (VI),
ou bien
on fait réagir le composé de formule (IX) avec un dérivé de formule généralé R'COY, dans laquelle R'CO correspond à un groupe R "acyle" tel que défini ci-dessus selon c) et Y représente un groupe labile tel qu'un atome d'halogène, par exemple le chlore, ou tel qu'un groupe imidazolyle-1, ou tel qu'un groupe alcoxy en $C_1$-$C_6$, ou tel qu'un groupe acyloxy de formule $R'CO_2$, obtenant ainsi un composé de formule générale (I) dans laquelle R représente un groupe "acyle" tel que défini ci-dessus, et si on le désire, on réduit le composé ainsi obtenu au moyen d'un hydrure simple ou complexe de bore, tel que le diborane ou le complexe de borane/sulfure de méthyle, l'hydrure de lithium et d'aluminium, l'hydrure d'aluminium, dans un solvant éthéré tel que l'éther diéthylique, le tétrahydrofuranne ou le dioxanne, à une température de 20 à 100°C, obtenant ainsi un composé de formule générale (I) dans laquelle R représente un groupe "réduit" tel que défini ci-dessus, ou bien
on soumet l'amine de formule (IX) à une alkylation au moyen d'un halogénure de formule générale RX (R étant un "réduit" tel que défini ci-desses et X un groupe labile), obtenant un composé de formule générale (I) où R est un groupe "réduit".

3. Procédé de préparation des composés selon la revendication 1, caractérisé en ce qu'on fait réagir la tétrahydro-1,2,3,4 isoquinoléine avec un tosylate de formule générale (XI)

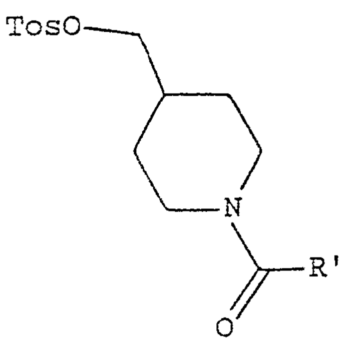

(XI)

(dans laquelle Tos représente un groupe tosyle et R'CO représente un groupe R "acyle"), soit en l'absence soit en présence d'un solvant inerte tel que le diméthylformamide ou le xyléne, à une température de 20 à 150°C, et éventuellement en présence d'une base organique telle qu'une amine tertiaire, ou minérale telle qu'un carbonate ou hydrogénocarbonate alcalin, obtenant ainsi un composé de formule générale (I) dans laquelle R représente un groupe "acyle" tel que défini ci-dessus, et si on le désire, on réduit le composé ainsi obtenu au moyen d'un hydrure simple ou complexe de bore, tel que le diborane ou le complexe de borane/sulfure de méthyle, l'hydrure de lithium et d'aluminium, l'hydrure d'aluminium, dans un solvant éthéré tel que l'éther diéthylique, le tétrahydrofuranne ou le dioxanne, à une température de 20 à 100°C, obtenant ainsi un composé de formule générale (I) dans laquelle R représente un groupe "réduit" tel que défini ci-dessus.

4. Procédé de préparation des composés selon la revendication 1, dans la formule générale desquels R représente un groupe "réduit", procédé caractérisé en ce qu'on fait réagir la tétrahydro-1,2,3,4 isoquinoléine avec un isonipécotate de formule générale (VIII)

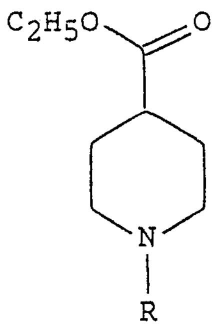

(VIII)

(dans laquelle R est un groupe "réduit"), en présence de triméthylaluminium, puis on réduit l'amide de formule générale (VII) ainsi obtenu

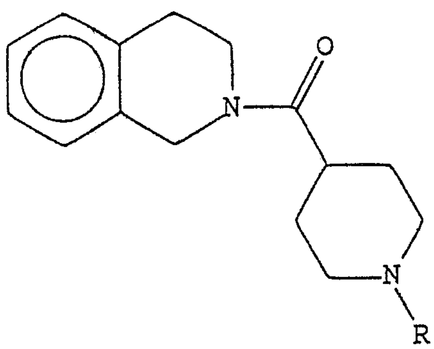

(VII)

au moyen d'hydrure de lithium et d'aluminium ou d'un hydrure simple ou complexe de bore, tel que le diborane ou le complexe de borane/sulfure de méthyle dans un solvant éthéré tel que l'éther diéthylique, le tétrahydrofuranne ou le dioxanne, à une température de 20 à 100°C.

5. Composition pharmaceutique caractérisée en ce qu'elle contient un composé selon la revendication 1.

**Revendications pour les états contractants : ES, GR**

1. Procédé de préparation de composés répondant à la formule générale (I)

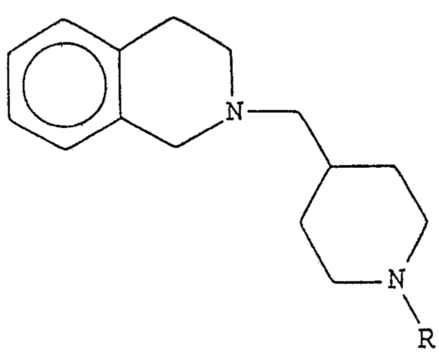

(I)

dans laquelle R représente

a) soit un atome d'hydrogène ;

b) soit un groupe ($C_1$-$C_6$)alkyle linéaire ou ramifié ; un groupe allyle ; un groupe ($C_3$-$C_6$)cycloalkylméthyle ; un groupe phénylméthyle portant éventuellement de un à trois substituants choisis parmi les atomes d'halogènes et les groupes trifluorométhyle, nitro, amino, diméthylamino, cyano, aminocarbonyle, ($C_1$-$C_3$)alkyle linéaires ou ramifiés, ($C_1$-$C_3$)alcoxy linéaires ou ramifiés, ($C_1$-$C_3$)alkylthio linéaires ou ramifiés ; un groupe phényl-2 éthyle ; un groupe phényl-3 propyle ; un groupe phényl-3 propène-2 yle ; un groupe phénylcarbonylméthyle ; un groupe naphtylméthyle ; un groupe pyridinylméthyle ; un groupe furannylméthyle ; ou un groupe thiénylméthyle ;

c) soit un groupe ($C_2$-$C_6$)alcanoyle linéaire ou ramifié ; un groupe ($C_3$-$C_6$)cycloalkylcarbonyle ; un groupe trifluoroacétyle ; un groupe phénylcarbonyle portant éventuellement de un à trois substituants choisis parmi les atomes d'halogènes et les groupes trifluorométhyle, nitro, ($C_1$-$C_3$)alkyle linéaires ou ramifiés, ($C_1$-$C_3$)alcoxy linéaires ou ramifiés, ($C_1$-$C_3$)alkylthio linéaires ou ramifiés ; un groupe oxo-1 phényl-3 propène-2 yle ; un groupe naphtylcarbonyle ; un groupe pyridinylcarbonyle ; un groupe furannylcarbonyle ; un groupe thiénylcarbonyle ; un groupe (indolyl-2)carbonyle ; ou un groupe (indolyl-5)carbonyle, procédé caractérisé en ce qu'on fait réagir la tétrahydro -1,2,3,4 isoquinoléine avec l'imidazolide de formule (III)

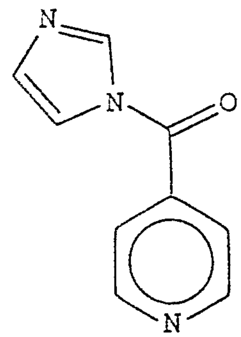

(III)

(préparé in situ au moyen d'acide isonicotinique et de N,N'-carbonyldiimidazole), obtenant ainsi l'amide de formule (IV)

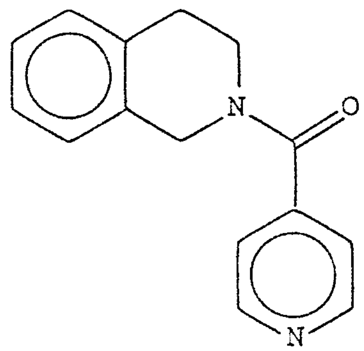

(IV)

que l'on soumet à une hydrogénation catalytique, donnant l'amide de formule (VI)

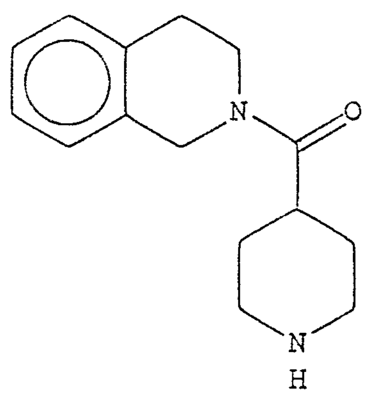

(VI)

qu'on réduite ou moyen d'hydrure de lithium et d'aluminium ou d'un hydrure simple ou complexe de bore, tel que le diborane ou le complexe de borane/sulfure de méthyle, obtenant ainsi le composé de formule (IX)

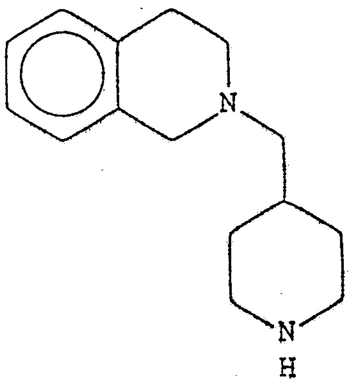

(IX)

qui correspond au composé de formule générale (I) dans laquelle R représente un atome d'hydrogène, ou bien

on soumet l'amide de formule (VI) à une alkylation au moyen d'un halogénure de formule générale RX dans laquelle R est un groupe "réduit" tel que défini ci-dessus selon b) et X est un groupe labile, par exemple un atome de brome, et finalement on réduit l'amide de formule générale (VII)

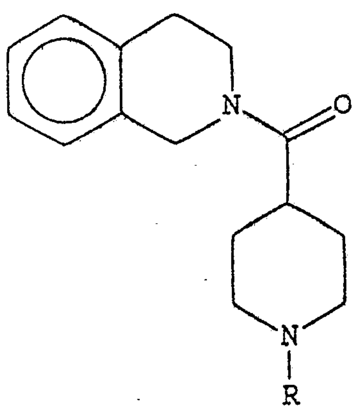

(VII)

ainsi obtenu de la manière décrite à propos de l'amide de formule (VI), ou bien

on fait réagir le composé de formule (IX) avec un dérivé de formule générale R'COY, dans laquelle R'CO correspond à un groupe R "acyle" tel que défini ci-dessus selon c) et Y représente un groupe labile tel qu'un atome d'halogène, par exemple le chlore, ou tel qu'un groupe imidazolyle-1, ou tel qu'un groupe alcoxy en $C_1$-$C_6$, ou tel qu'un groupe acyloxy de formule $R'CO_2$, obtenant ainsi un composé de formule générale (I) dans laquelle R représente un groupe "acyle" tel que défini ci-dessus, et si on le désire, on réduit le composé ainsi obtenu au moyen d'un hydrure simple ou complexe de bore, tel que le diborane ou le complexe de borane/sulfure de méthyle, l'hydrure de lithium et d'aluminium, l'hydrure d'aluminium, dans un solvant éthéré tel que l'éther diéthylique, le tétrahydrofuranne ou le dioxanne, à une température de 20 à 100°C, obtenant ainsi un composé de formule générale (I) dans laquelle R représente un groupe "réduit" tel que défini ci-dessus, ou bien

on soumet l'amine de formule (IX) à une alkylation au moyen d'un halogénure de formule générale RX (R étant un "réduit" tel que défini ci-dessus et X un groupe labile), obtenant un composé de formule générale (I) où R est un groupe "réduit".

2. Procédé de préparation de composés répondant à la formule générale (I)

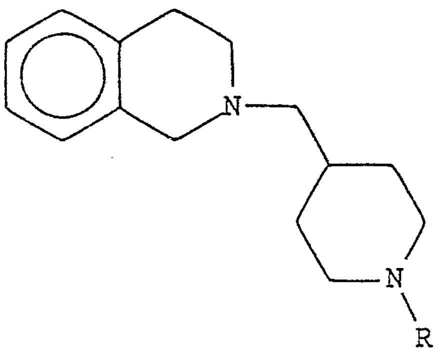

(I)

dans laquelle R représente
- soit un groupe ($C_1$-$C_6$)alkyle linéaire ou ramifié ; un groupe allyle ; un groupe ($C_3$-$C_6$)cycloalkylméthyle ; un groupe phénylméthyle portant éventuellement de un à trois substituants choisis parmi les atomes d'halogènes et les groupes trifluorométhyle, nitro, amino, diméthylamino, cyano, aminocarbonyle, ($C_1$-$C_3$)alkyle linéaires ou ramifiés, ($C_1$-$C_3$)alcoxy linéaires ou ramifiés, ($C_1$-$C_3$)alkylthio linéaires ou ramifiés ; un groupe phényl-2 éthyle ; un groupe phényl-3 propyle ; un groupe phényl-3 propène-2 yle ; un groupe phénylcarbonylméthyle ; un groupe naphtylméthyle ; un groupe pyridinylméthyle ; un groupe furannylméthyle ; ou un groupe thiénylméthyle ;
- soit un groupe ($C_2$-$C_6$)alcanoyle linéaire ou ramifié ; un groupe ($C_3$-$C_6$)cycloalkylcarbonyle ; un groupe trifluoroacétyle ; un groupe phénylcarbonyle portant éventuellement de un à trois substituants choisis parmi les atomes d'halogènes et les groupes trifluorométhyle, nitro, ($C_1$-$C_3$)alkyle linéaires ou ramifiés, ($C_1$-$C_3$)alcoxy linéaires ou ramifiés, ($C_1$-$C_3$)alkylthio linéaires ou ramifiés ; un groupe oxo-1 phényl-3 propène-2 yle ; un groupe naphtylcarbonyle ; un groupe pyridinylcarbonyle ; un groupe furannylcarbonyle ; un groupe thiénylcarbonyle ; un groupe (indolyl-2)carbonyle ; ou un groupe (indolyl-5)carbonyle,
procédé caractérisé en ce qu'on fait réagir la tétrahydro-1,2,3,4 isoquinoléine avec un tosylate de formule générale (XI)

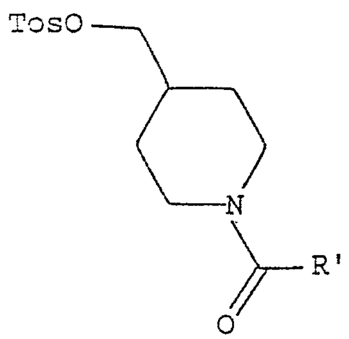

(XI)

(dans laquelle Tos représente un groupe tosyle et R′CO représente un groupe R "acyle"), soit en l'absence soit en présente d'un solvant inerte tel que le diméthylformamide ou le xyléne, à une température de 20 à 150° C, et éventuellement en présence d'une base organique telle qu'une amine tertiaire, ou minérale telle qu'un carbonate ou hydrogénocarbonate alcalin, obtenant ainsi un composé de formule générale (I) dans laquelle R représente un groupe "acyle" tel que défini ci-dessus, et si on le désire, on réduit le composé ainsi obtenu au moyen d'un hydrure simple ou complexe de bore, tel que le diborane ou le complexe de borane/sulfure de methyle, l'hydrure de lithium et d'aluminium, l'hydrure d'aluminium, dans un solvant éthéré tel que l'éther diéthylique, le tétrahydrofuranne ou le dioxanne, à une température de 20 à 100° c,obtenant ainsi un composé de formule générale (I) dans laquelle R représente un groupe "réduit" tel que défini ci-dessus.

3. Procédé de préparation de composés répondant à la formule générale (I)

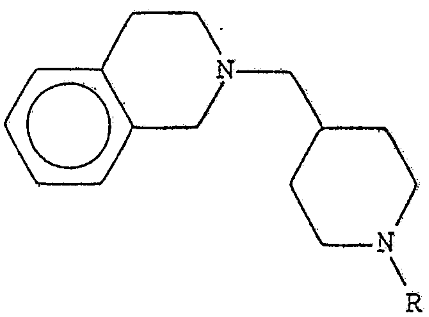

(I)

dans laquelle R représente
a) soit un atome d'hydrogène ;
b) soit un groupe ($C_1$-$C_6$)alkyle linéaire ou ramifié ; un groupe allyle ; un groupe ($C_3$-$C_6$)cycloalkylméthyle ; un groupe phénylméthyle portant éventuellement de un à trois substituants choisis parmi les atomes d'halogènes et les groupes trifluorométhyle, nitro, amino, diméthylamino, cyano, aminocarbonyle, ($C_1$-$C_3$)alkyle linéaires ou ramifiés, ($C_1$-$C_3$)alcoxy linéaires ou ramifiés, ($C_1$-$C_3$)alkylthio linéaires ou ramifiés ; un groupe phényl-2 éthyle ; un groupe phényl-3 propyle ; un groupe phényl-3 propène-2 yle ; un groupe phénylcarbonylméthyle ; un groupe naphtylméthyle ; un groupe pyridinylméthyle ; un groupe furannylméthyle ; ou un groupe thiénylméthyle,
procédé caractérisé en ce qu'on fait réagir la tétrahydro-1,2,3,4 isoquinoléine avec un isonipécotate de formule générale (VIII)

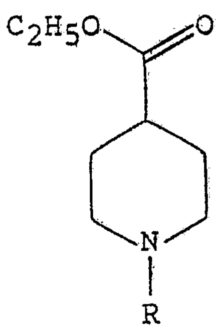

(VIII)

(dans laquelle R est un groupe "réduit"), en présence de triméthylaluminium, puis on réduit l'amide de formule générale (VII) ainsi obtenu

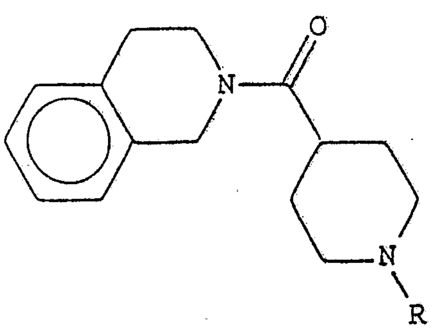

(VII)

au moyen d'hydrure de lithium et d'aluminium ou d'un hydrure simple ou complexe de bore, tel que le diborane ou le complexe de borane/sulfure de méthyle dans un solvant éthéré tel que l'éther diéthylique, le tétrahydrofuranne ou le dioxanne, à une température de 20 à 100°C.

Office européen des brevets

# RAPPORT DE RECHERCHE EUROPEENNE

Numero de la demande

EP 88 40 2022

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 035 063 (SARATH)<br>* Page 1, ligne 1 - page 2, ligne 3; revendications 1,14 *<br>--- | 1,5 | C 07 D 401/06<br>C 07 D 401/14<br>C 07 D 405/14<br>C 07 D 409/14<br>A 61 K 31/47 |
| A | FR-A-1 580 180 (AMERICAN HOME PRODUCTS)<br>* Page 1, colonne 1, lignes 1-27; page 4, colonne 1, lignes 52-58 *<br>--- | 1,5 | |
| A | FR-M- 7 607 (ROUSSEL-UCLAF)<br>* Page 1, lignes 1-9 *<br>----- | 1,5 | |
| | | | **DOMAINES TECHNIQUES RECHERCHES (Int. Cl.4)** |
| | | | C 07 D 401/00<br>C 07 D 405/00<br>C 07 D 409/00<br>C 07 D 217/00<br>C 07 D 211/00<br>C 07 D 209/00<br>A 61 K 31/00 |

Le présent rapport a été établi pour toutes les revendications

| Lieu de la recherche | Date d'achèvement de la recherche | Examinateur |
|---|---|---|
| LA HAYE | 15-11-1988 | BRENNAN J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)